# EUROPEAN PATENT APPLICATION

(11) **EP 0 807 626 A1**
(43) Date of publication of application: **19.11.1997**
(21) Application number: 96901514.8
(22) Date of filing: 01.02.1996
(51) Int. Cl.: C07D 213/42, A61K 31/44

(54) **N-HYDROXYUREA DERIVATIVES**

(30) Priority: 02.02.1995 JP 35858/95; 07.04.1995 JP 107084/95
(71) Applicant: Nikken Chemicals Company, Limited, Tokyo 104 (JP)
(72) Inventor: KOMATSU, Toshiya, Saitama 336 (JP); KAWAHARA, Yoshikazu, Gunma 370 (JP); FUJII, Tsutomu, Saitama 335 (JP); TAKANO, Michika, Saitama 336 (JP); HARA, Norie, Tokyo 183 (JP)
(74) Representative: Kador & Partner
(86) International application number: JP9600204
(87) International publication number: WO9623772

(57) **Abstract**

An N-hydroxyurea derivative or a pharmacologically acceptable salt thereof having the formula (I): 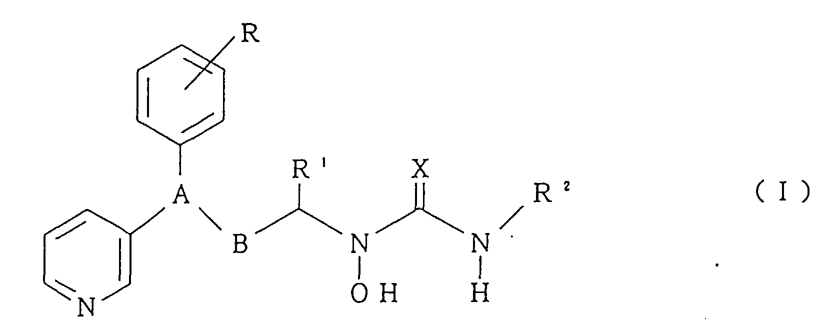 wherein, A represents CH, CH-CH₂, CH-O, or C=CH, X represents O or S, B represents a C₁ to C₈ alkylene, C₂ to C₆ alkenylene, m-phenylene, or p-phenylene, R represents a hydrogen atom, halogen atom, C₁ to C₄ alkyl which may be substituted with a halogen atom, C₁ to C₄ alkoxy which may be substituted with a halogen atom, or nitro, R¹ represents a hydrogen atom, C₁ to C₄ lower alkyl, or phenyl, and R² represents a hydrogen atom, C₁ to C₄ alkyl, cycloalkyl, phenyl which may be substituted with a substituent, or methanesulfonyl.

## Description

### TECHNICAL FIELD

The present invention relates to novel N-hydroxyurea derivatives, which have a lipoxygenase inhibiting action and a thromboxane synthase inhibiting action, and therefore, are useful in treating or alleviating allergic diseases or inflammatory diseases.

### BACKGROUND ART

In recent years, the roles of chemical mediators in asthma and other allergic diseases have been elucidated. In addition to histamine, PAF, leukotrienes, thromboxane, etc. have become known. It has been shown that leukotrienes are biosynthesized by the activity of 5-lipoxygenase from arachidonic acid, and thromboxane A₂ is biosynthesized by thromboxane synthase after the catabolism with cyclooxygenase from arachidonic acid. Further, both leukotrienes and thromboxane A₂ have been found to be important chemical mediators in allergic reactions, which cause various diseases such as asthma, psoriasis, enteritis, nephritis, ulcers, and ischemia. As a compounds which inhibit thromboxane synthase, for example, imidazole derivatives are known (DE-A-2923815). On the other hand, as compounds which inhibit 5-lipoxygenase, for example, benzothiophene derivatives are known (EP-A-279263). However, if it-were possible to inhibit both thromboxane synthase and 5-lipoxygenase, a greater effect in treating or alleviating the above-mentioned diseases could be given than with the known compounds for inhibition to single enzymes.

Recently, as compounds for inhibiting the biosynthesis of such two mediators, quinone derivatives (see Japanese Unexamined Patent Publication (Kokai) No. 63-45257, Japanese Unexamined Patent Publication (Kokai) No. 5-78321), chroman derivatives or dihydrobenzofuran derivatives (Japanese Unexamined Patent Publication (Kokai) No. 5-310724), imidazolylphenol derivatives (Japanese Unexamined Patent Publication (Kokai) No. 6-9571), and aminocoumaran derivatives (Japanese Unexamined Patent Publication (Kokai) No. 6-41123) have become known.

### DISCLOSURE OF INVENTION

An object of the present invention is to provide compounds capable of inhibiting the biosynthesis of both leukotrienes and thromboxane A₂, that is, novel compounds having the dual inhibition against lipoxygenase and thromboxane synthase at the same time.

In accordance with the present invention, there is provided an N-hydroxyurea derivative or a pharmacologically acceptable salt thereof having the formula (I): 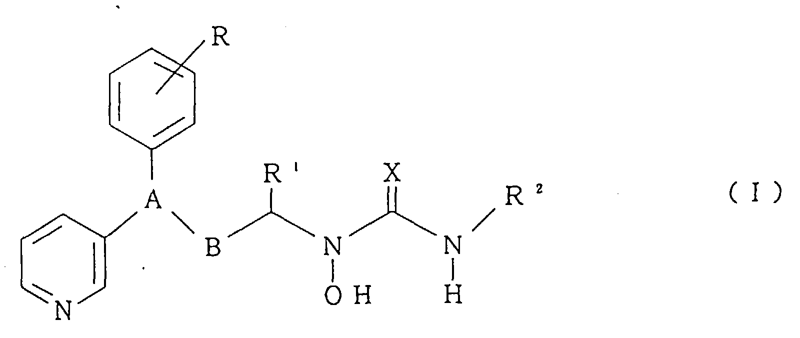 wherein, A represents CH, CH-CH₂, CH-O, or C=CH, X represents O or S, B represents a C₁ to C₈ alkylene, C₂ to C₆ alkenylene, m-phenylene, or p-phenylene, R represents a hydrogen atom, halogen atom, C₁ to C₄ alkyl which may be substituted with a halogen atom, C₁ to C₄ alkoxy which may be substituted with a halogen atom, or nitro, R¹ represents a hydrogen atom, C₁ to C₄ lower alkyl, or phenyl, and R² represents a hydrogen atom, C₁ to C₄ alkyl, cycloalkyl, phenyl which may be substituted with, or methanesulfonyl.

In accordance with the present invention, there is also provided a pharmaceutical composition containing the above-mentioned derivative or a pharmacologically acceptable salt thereof, as an active ingredient, and with a carrier, preferably a pharmaceutical preparation for treatment of allergic diseases or inflammatory diseases, particularly preferably an antiasthmatic pharmaceutical preparation.

### BEST MODE FOR CARRYING OUT THE INVENTION

The present invention will be explained in further detail below.

In the N-hydroxyurea derivatives represented by the formula (I), as preferable combinations of A and B, when A represents CH, B represents a C₁ to C₈ alkylene, when A represents CH-CH₂ or CH-O, B represents a C₂ to C₆, preferably C₂ to C₄, alkenylene, m-phenylene, or p-phenylene, and when A represents C=CH, B represents a C₁ to C₈, preferably a C₁ to C₅, alkylene. Further, as the R, R¹, and R², C₁ to C₄ lower alkyl, a methyl, ethyl, isopropyl, butyl, etc. may be mentioned. As the methyl substituted with a halogen atom for R, a trifluoromethyl, difluoromethyl, etc. may be mentioned. As the C₁ to C₄ lower alkoxy for R, a methoxy, ethoxy, propoxy, etc. may be mentioned. As the cycloalkyl for R², a cyclohexyl, cyclopentyl, cycloheptyl, etc. may be mentioned. As the substitutable phenyl for R², a phenyl and phenyl substituted with a methyl, ethyl, methoxy, ethoxy, halogen atom, etc. may be mentioned.

Next, the preferable groups of the N-hydroxyurea derivative shown in formula (I) will be shown. As a preferable group of R, a hydrogen atom, fluorine atom, isopropyl, methoxy, or trifluoromethyl, in particular, a hydrogen atom may be mentioned. As a preferable group of R¹, a hydrogen atom or C₁ to C₄ lower alkyl, particularly, a hydrogen atom or methyl may be mentioned. As a preferable group of R², a hydrogen atom, ethyl, isopropyl, cyclohexyl, phenyl, or methoxyphenyl, particularly, a hydrogen atom or cyclohexyl, may be mentioned.

As preferable compounds of the present invention, all of the compounds synthesized in the Examples (Compound 1 to Compound 48) may be mentioned. Among these compounds, the following compounds are particularly preferable:
(Compound 1) N-hydroxy-N-[(E)-7-phenyl-7-(3-pyridyl)-6-hepten-1-yl]urea
(Compound 2) N-hydroxy-N-[(Z)-7-phenyl-7-(3-pyridyl)-6-hepten-1-yl]urea
(Compound 3) N'-cyclohexyl-N-hydroxy-N-[(Z)-7-phenyl-7-(3-pyridyl)-6-hepten-1-yl]urea
(Compound 4) N-hydroxy-N-[4-(3-pyridyl)-4-(3-trifluoromethylphenyl)-3-buten-1-yl]urea
(Compound 7) N-hydroxy-N-[4-phenyl-4-(3-pyridyl)butyl]urea
(Compound 9) N'-cyclohexyl-N-hydroxy-N-[4-phenyl-4-(3-pyridyl)butyl]urea
(Compound 10) N'-cyclohexyl-N-hydroxy-N-[4-phenyl-4-(3-pyridyl)butyl]thiourea
(Compound 11) N-hydroxy-N'-(3-methoxyphenyl)-N-[4-phenyl-4-(3-pyridyl)butyl]urea
(Compound 17) N-hydroxy-N-[5-phenyl-5-(3-pyridyl)pentyl]urea
(Compound 20) N-hydroxy-N-[6-phenyl-6-(3-pyridyl)hexyl]urea
(Compound 22) N-hydroxy-N-[6-phenyl-6-(3-pyridyl)hexyl]thiourea
(Compound 23) N'-ethyl-N-hydroxy-N-[6-phenyl-6-(3-pyridyl)hexyl]urea
(Compound 24) N-hydroxy-N-[7-phenyl-7-(3-pyridyl)heptyl]urea
(Compound 25) N'-cyclohexyl-N-hydroxy-N-[7-phenyl-7-(3-pyridyl)heptyl]urea
(Compound 26) N-hydroxy-N'-(3-methoxyphenyl)-N-[7-phenyl-7-(3-pyridyl)heptyl]urea
(Compound 28) N-hydroxy-N-[1-methyl-7-phenyl-7-(3-pyridyl)heptyl]urea
(Compound 29) N-hydroxy-N'-phenyl-N-[8-phenyl-8-(3-pyridyl)octyl]thiourea
(Compound 32) N'-cyclohexyl-N-hydroxy-N-[1-methyl-9-phenyl-9-(3-pyridyl)nonyl]thiourea
(Compound 33) N-hydroxy-N-[10-phenyl-10-(3-pyridyl)decyl]urea
(Compound 40) N-hydroxy-N-[(E)-7-phenyl-7-(3-pyridyl)-2-hepten-1-yl]urea
(Compound 41) N-hydroxy-N-[(E)-1-methyl-7-phenyl-7-(3-pyridyl)-2-hepten-1-yl]urea
(Compound 42) N-hydroxy-N-[3-[α-(3-pyridyl) benzyloxy]benzyl]urea
(Compound 43) N-hydroxy-N-[4-[α-(3-pyridyl) benzyloxy]benzyl]urea
(Compound 44) N'-cyclohexyl-N-hydroxy-N-[3-[α-(3-pyridyl) benzyloxy]benzyl]urea
(Compound 45) N-hydroxy-N'-(3-methoxyphenyl)-N-[3-[α-(3-pyridyl) benzyloxy]benzyl]urea
(Compound 46) N-hydroxy-N-[3-[α-(3-pyridyl) benzyloxy]-α-methylbenzyl]urea
(Compound 47) N-hydroxy-N-[3-[β-(3-pyridyl) phenethyl] benzyl]urea
(Compound 48) N-hydroxy-N-[3-[β-(3-pyridyl) phenethyl]-α-methylbenzyl]urea

Among the N-hydroxyurea derivatives having the formula (I), compounds where A is CH-CH₂, CH-O, or CH have at least one asymmetric carbon atom and exist as an enantiomer or a number of diastereomers. The present invention includes all of these isomers and isomeric mixtures. Further, if necessary, the fractional crystallization method, chromatography, etc. may be used to separate an isomeric mixture into the respective isomers. On the other hand, in the case of a compound having a carbon-carbon double bond in the molecule, there are two geometrical isomers. The present invention includes these isomers and isomeric mixture. Further, if necessary, the fractional crystallization method, chromatography, etc. may be used to separate the isomeric mixture into the respective geometrical isomers.

In the present invention, the N-hydroxyurea derivative having the formula (I) may also be a pharmacologically acceptable salt. Specifically, an inorganic salts formed from hydrochloric acid, hydrobromic acid, sulfuric acid, bisulfurous acid, phosphoric acid, etc. and organic salts formed from formic acid, acetic acid, citric acid, fumaric acid, gluconic acid, lactic acid, maleic acid, succinic acid, tartaric acid, methanesulfonic acid, benzenesulfonic acid, toluenesulfonic acid, etc. may be mentioned.

The compounds according to the present invention may be produced by many methods. The compounds where A is C=CH, for example, the Compound 1, is synthesized according to the following reaction process. The other compounds may also be synthesized with reference to this reaction process.

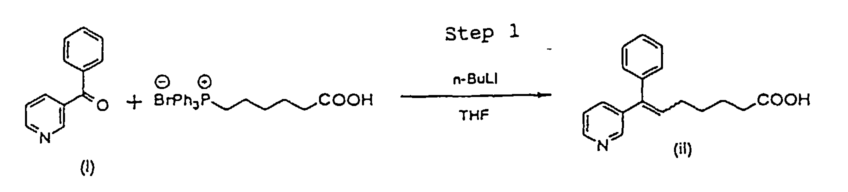

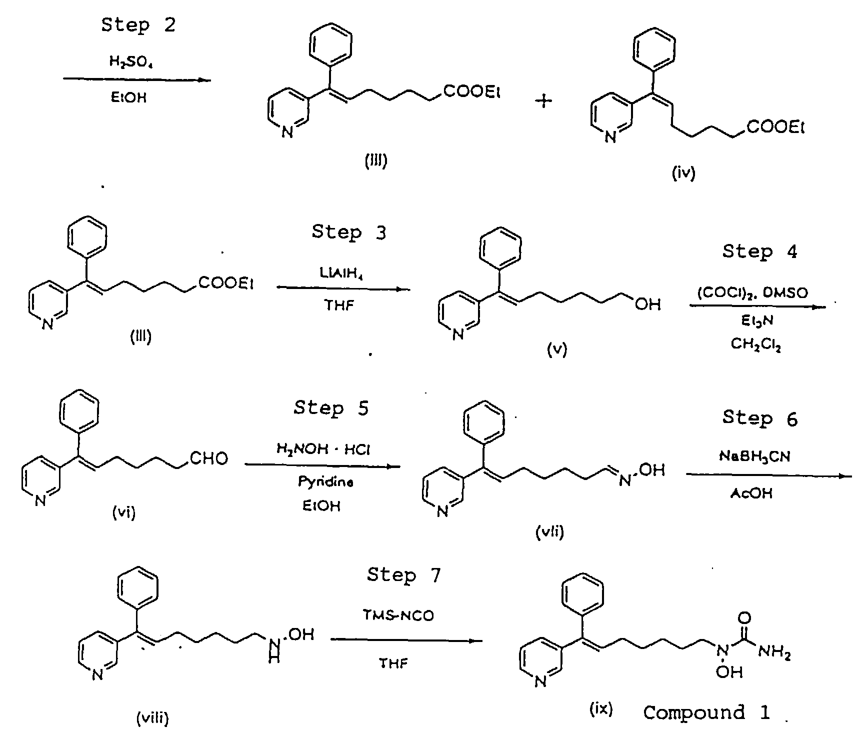

The starting substances used in the above reaction process are commercially available compounds or compounds can be produced from known compounds by known methods.
Step 1: First, carboxylic acid (ii) is synthesized from 3-benzoylpyridine(i) by a known standard method (Wittig reaction).
Step 2: From the carboxylic acid (ii), the esters (iii) and (iv) are synthesized by known methods.
Step 3: The ester (iii) is converted to alcohol (v) by using various types of reducing agents, for example, lithium aluminum hydride.
Step 4: The alcohol (v) is oxidized to synthesize an aldehyde (vi). The oxidation reaction used at this stage may be performed, for example, by the method of D. Swern et al. (see the Journal of Organic Chemistry, vol. 43, p. 2480, 1978).
Step 5: The aldehyde (vi) is reacted with hydroxylamine hydrochloride to synthesize an oxime (vii).
Step 6: The oxime (vii) is reduced by a reducing agent such as sodium cyanoborohydride to obtain a hydroxylamine (viii).
Step 7: The hydroxylamine (viii) is reacted with trimethylsilylisocyanate to obtain the Compound 1 (ix).
   Further, among the compounds where A is C=CH, the compounds where R is the substituent other than a hydrogen atom may be produced by a different method. For example, the reaction process is shown below taking, as an example, the Compound 4. The starting substances used in the above reaction process are commercially available compounds or compounds produced from known compounds by known methods. 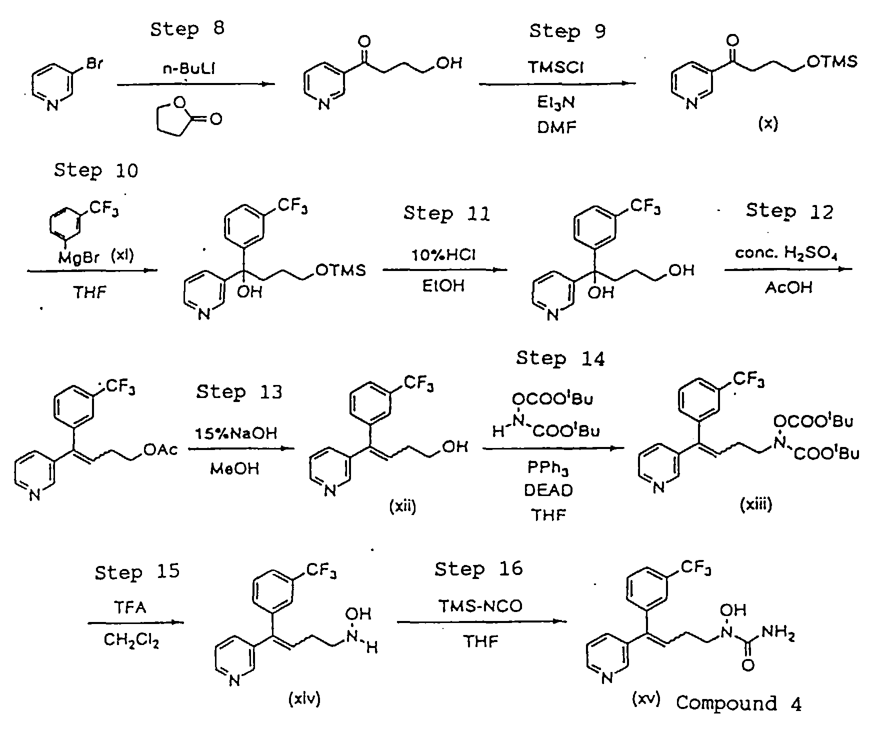
Steps 8 to 13: First, the known method of S. Ohkawa et al. (see the Journal of Medicinal Chemistry, vol. 34, p. 267, 1991) is used, that is, 3-bromopyridine is used as a starting material and is acylated (step 8) and trimethylsilylated (TMS) (step 9) to synthesize a ketone (x). A Grignard reagent (xi) is added to this (x) (step 10), the protective groups are removed (step 11), dehydrogenation is performed (step 12), and then deacetylation is performed (step 13) to derive the alcohol (xii).
Steps 14 and 15: At this stage, from (xii), conditions of Mitsunobu reaction are used to cause a reaction with N,O-bis(tert-butoxycarbonyl)hydroxylamine to synthesize an N,O-protected hydroxylamine (xiii). Next, acid hydrolysis is performed to derive hydroxylamine (xiv) (see WO 92/01682).
Step 16: At this stage, the same method as in step 7 of the synthesis of the Compound 1 is used to obtain the desired product of the Compound 4 (xv).
   Further, among the compounds where A is CH, for example, the Compound 5 is synthesized in accordance with the following reaction process. The other compounds may also be synthesized with reference to this process. 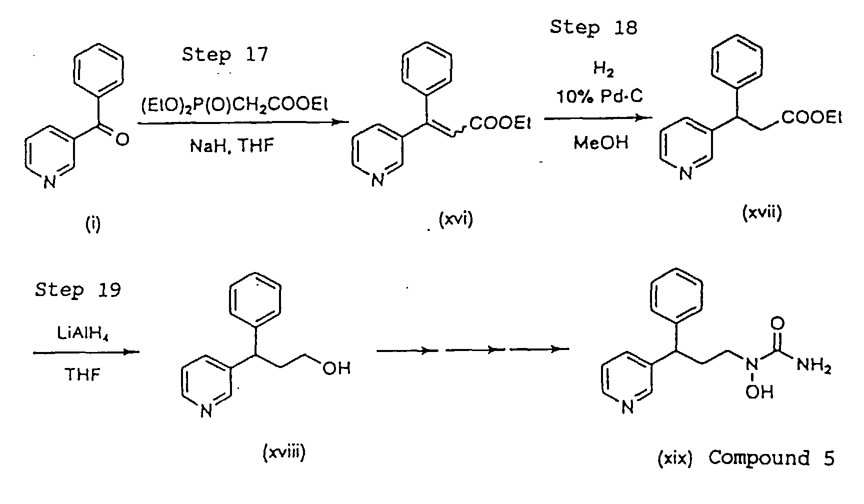
   The starting substances used in the above reaction process are commercially available compounds or compounds can be produced from known compounds by known methods.
Step 17: First, from 3-benzoylpyridine (i), a known standard method (Horner-Emmons reaction) is used to synthesize an α,β-unsaturated ester (xvi). The reaction may be performed in a range of -78°C to room temperature, but -30°C to -10°C is the most preferable reaction temperature.
Step 18: From the α,β-unsaturated ester (xvi), a known method, for example, a catalytic hydrogenation reaction using a catalyst such as activated carbon with palladium, is used to synthesize an ester (xvii).
Step 19: The ester (xvii) is reduced by various types of reducing agents, such as lithium aluminum hydride, to produce an alcohol (xviii).
   Compound 5 (xix) can be obtained by the same reaction as with steps 14 to 16 of the synthesis of the Compound 4.
   Further, among the compounds where A is CH, for example, the Compound 30 is synthesized according to the following reaction process. The other compounds may also be synthesized with reference to this process. 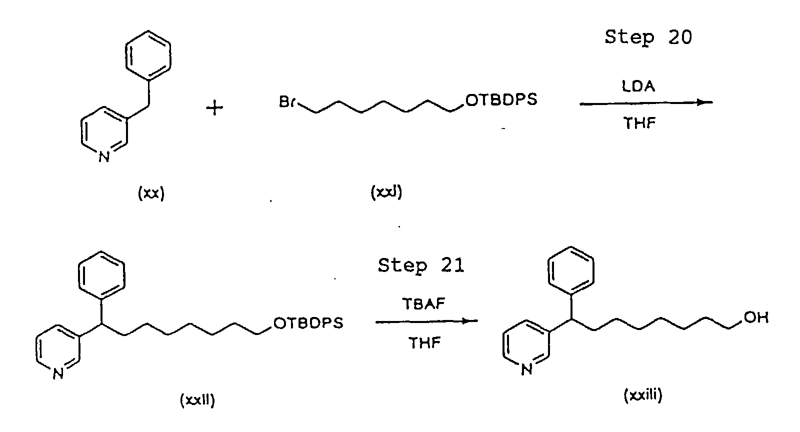 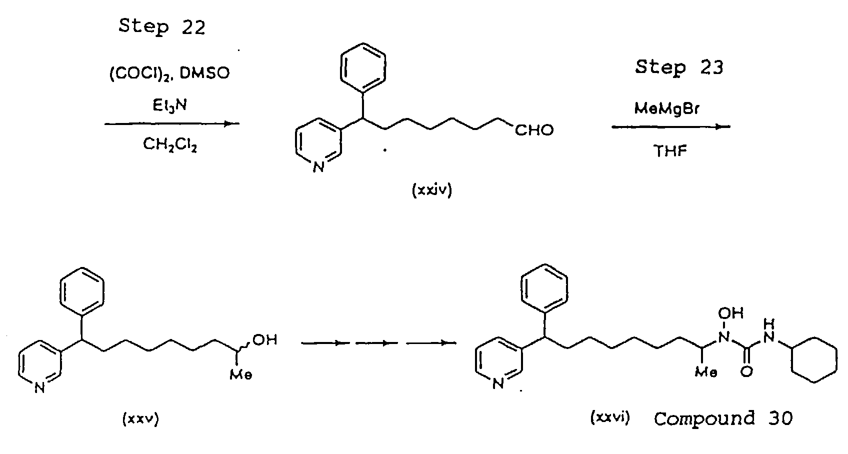
   The starting substances used in the above reaction process are commercially available compounds or compounds can be produced from known compounds by known methods.
Step 20: First, 3-benzylpyridine (xx) is subjected to a condensation reaction with 7-t-butyldiphenylsilyloxyheptyl-1-bromide (xxi) to obtain a silyl compound (xxii). For this reaction, a strong base such as lithium diisopropylamide (LDA) is used.
Step 21: At this step, the silyl compound (xxii) is converted to an alcohol (xxiii). The reaction is preferably performed by a method using tetrabutylammonium fluoride (TBAF).
Step 22: From this alcohol (xxiii), the same reaction as in step 4 in the synthesis of the Compound 1 is used to synthesize an aldehyde (xxiv).
Step 23: At this step, the aldehyde (xxiv) is reacted with methylmagnesium bromide (MeMgBr) etc. to synthesize an alcohol (xxv).
   Compound 30 (xxvi) is obtained by the same reaction as in steps 14 to 16 of the synthesis of the Compound 4 (using, instead of trimethylsilylisocyanate, cyclohexylisocyanate).
   Further, among the compounds where A represents CH-CH₂ or CH-O and B represents a C₂ to C₆ alkylene group, m-phenylene group, or p-phenylene group, for example, the Compound 46 may be synthesized according to the following reaction process. The other compounds may be synthesized with reference to this process. 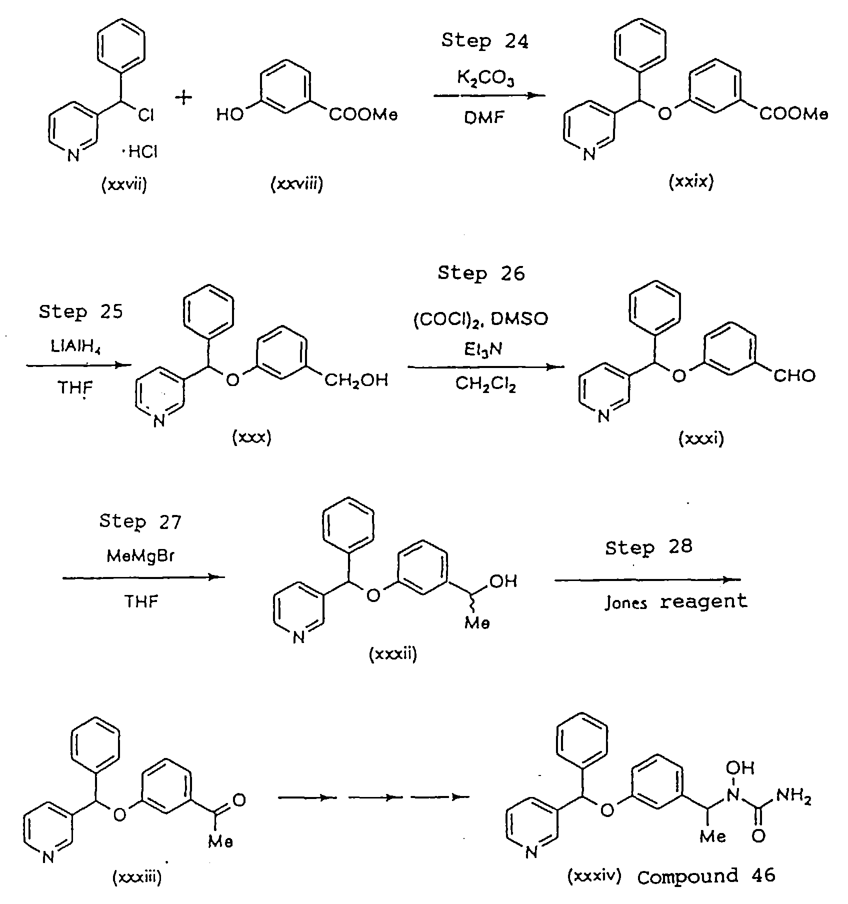
   The starting substances used in the above reaction process are commercially available compounds or compounds which can be produced from known compounds by known methods.
Step 24: Chloride (xxvii) and m-hydroxybenzoate methyl ester (xxviii) are react in the presence of a base (for example, potassium carbonate or sodium hydride) to synthesize an ester (xxix).
Steps 25 and 26: The ester (xxix) was subjected to the same reaction as step 3 and 4 in the synthesis of the Compound 1 to form an alcohol (xxx) and then an aldehyde (xxxi).
Step 27: The aldehyde (xxxi) was subjected to the same reaction as in step 23 for the synthesis of the Compound 30 to synthesize an alcohol (xxxii).
Step 28: From the alcohol (xxxii), a ketone (xxxiii) was synthesized. The oxidation reaction used may, for example, be by the method of E. R. H. Jones et al. (see the Journal of Chemical Society, p. 2548, 1953).

After this, the same reaction as in steps 5 to 7 of the synthesis of the Compound 1 may be used to obtain the desired final Compound 46 (xxxiv).

The pharmacologically acceptable salts of the compounds according to the present invention may be simply prepared by a reaction of the compounds of the present invention with the above-mentioned nontoxic inorganic acids or organic acids by an ordinary method. When the compounds according to the present invention are used as anti-allergic or antiinflammatory, they may be administered by a suitable oral or non-oral method of administration.

As a form of oral administration, tablets, granules, capsules, pills, powders, etc. may be exemplified. Further as a form of non-oral administration, injections, inhalants, suppositories, solutions, etc. may be exemplified. When preparing these medically administered compounds, the compounds according to the present invention or the salts thereof may be prepared in accordance with ordinary methods.

For example, in the case of oral administration, the preparations can be prepared into the desired form using excipients such as lactose, glucose, corn starch, sucrose, disintegrators such as calcium carboxymethylcellulose, hydroxypropylcellulose, lubricants such as calcium stearate, magnesium stearate, talc, polyethylene glycol, hardened oil, binding agents such as hydroxypropylcellulose, hydroxypropylmethylcellulose, carboxymethylcellulose, polyvinyl alcohol, gelatin, gum arabic, humectants such as glycerine, ethylene glycol, and, in addition, if necessary, surfactants, corrigents, etc.

Further, in the case of a non-oral drug, diluents such as water, ethanol, glycerine, propylene glycol, polyethylene glycol, agar, tragacanth gum, may be used and, if necessary, solubilization aids, buffer agents, preservatives, flavors, coloring agents, etc. may be used.

When formulating the compounds of the present invention as anti-allergic, the dosage, as the compound of the present invention, is per adult, in the case of oral administration, 1 to 3000 mg per day, preferably 30 to 800 mg, and in the case of non-oral administration, 0.5 to 500 mg per day, preferably 1 to 50 mg. The desired effect of treatment can be expected by administration divided into one to three dosages per day.

### EXAMPLES

The Synthesis Examples, Preparation Examples, and Test Examples of the compound according to the present invention will be exemplified below, but the present invention is of course not limited to these examples:

### Synthesis Examples

### Example 1

### Synthesis of N-hydroxy-N-[(E)-7-phenyl-7-(3-pyridyl)-6-hepten-1-yl]urea (Compound 1)

### (1) (E,Z)-7-Phenyl-7-(3-pyridyl)-6-heptenoic acid

Under an argon atmosphere (under Ar), 5-carboxypentyltriphenyl phosphonium bromide (31.00 g, 67.83 mmol) was suspended in tetrahydrofuran (300 ml), followed by dropwise adding n-butyl lithium (1.47 M, n-hexane solution, 92.30 ml, 135.66 mmol) at 0°C. The mixture was then stirred at the same temperature for 20 minutes. Next, a tetrahydrofuran (50 ml) solution of 3-benzoylpyridine (10.30 g, 56.53 mmol) was dropwise added at 0°C and the mixture stirred at room temperature for 2 hours. After the reaction, the reaction mixture was extracted twice with water (200 ml), and a pH of the aqueous layer was adjusted to 6 with a 10% aqueous hydrochloric acid solution, followed by extracting with ethyl acetate. The organic phase was washed with brine and dried over magnesium sulfate, then the solvent was evaporated in vacuo to obtain (E,Z)-7-phenyl-7-(3-pyridyl)-6-heptenoic acid (18.74 g) as a white crystal.

### (2) (E)-7-Phenyl-7-(3-pyridyl)-6-heptenoate ethyl ester and (Z)-7-phenyl-7-(3-pyridyl)-6-heptenoate ethyl ester

Concentrated sulfuric acid (40 ml) was dropwise added to an ethanol (200 ml) solution of (E,Z)-7-phenyl-7-(3-pyridyl)-6-heptenoic acid (18.74 g). The mixture was heated at reflux for 3 hours. After the reaction, the solution was returned to room temperature, was made a pH of 8 to 9 with a saturated aqueous sodium hydrogencarbonate solution, and was extracted with ethyl acetate. The organic phase was washed with brine and dried over magnesium sulfate, then the solvent was evaporated in vacuo and the residue was purified by silica gel column chromatography to obtain (E)-7-phenyl-7-(3-pyridyl)-6-heptenoate ethyl ester (5.61 g) and (Z)-7-phenyl-7-(3-pyridyl)-6-heptenoate ethyl ester(4.60 g) as white crystals.

### (3) (E)-7-Phenyl-7-(3-pyridyl)-6-hepten-1-ol

Under Ar, lithium aluminum hydride (123 mg, 3.24 mmol) was added to a tetrahydrofuran (20 ml) solution of (E)-7-phenyl-7-(3-pyridyl)-6-heptenoate ethyl ester (1.00 g, 3.24 mmol) at 0°C and the mixture was stirred at room temperature for 1 hour. After the reaction, water (5.0 ml) was added, then the mixture was stirred at room temperature for 15 minutes, then diluted by tetrahydrofuran (10 ml) and passed through a pad of Celite. The filtrate was diluted with ethyl acetate, then was washed with brine and dried over magnesium sulfate, then the solvent was evaporated in vacuo to obtain (E)-7-phenyl-7-(3-pyridyl)-6-hepten-1-ol (0.95 g) as a yellow oily substance.

### (4) (E)-7-Phenyl-7-(3-pyridyl)-6-heptenal

Under Ar, dimethylsulfoxide (1.01 ml, 14.24 mmol) was dropwise added at -78°C to a dichloromethane (40 ml) solution of oxalyl chloride (0.61 ml, 7.12 mmol) and the mixture was stirred for 30 minutes. Next, at the same temperature, a dichloromethane (10 ml) solution of (E)-7-phenyl-7-(3-pyridyl)-6-hepten-1-ol (0.95 g) was dropwise added and the mixture was stirred at -78°C for 1 hour. Further, triethylamine (2.97 ml, 21.37 mmol) was added and the mixture was stirred at room temperature for 1.5 hours. After the reaction, water was dropwise added, then the mixture was stirred at room temperature for 30 minutes, then extracted with ether. The organic phase was washed with an aqueous saturated sodium hydrogencarbonate solution and brine successively and dried over magnesium sulfate. Then the solvent was evaporated in vacuo and the residue was purified by silica gel column chromatography to obtain (E)-7-phenyl-7-(3-pyridyl)-6-heptenal (0.83 g) as a yellow oily substance.

### (5) (E)-7-Phenyl-7-(3-pyridyl)-6-heptenal oxime

Pyridine (2.0 ml) and hydroxylamine hydrochloride (0.33 g, 4.72 mmol) were added to an ethanol (2.0 ml) solution of (E)-7-phenyl-7-(3-pyridyl)-6-heptenal (0.83 g, 3.15 mmol), then the mixture was stirred at room temperature for 2 hours. After the reaction, the solvent was concentrated in vacuo, the resultant mixture was diluted with ethyl acetate, then washed with water and brine successively. The organic phase was dried over magnesium sulfate, then the solvent was evaporated in vacuo and the residue was purified by silica gel column chromatography to obtain (E)-7-phenyl-7-(3-pyridyl)-6-heptenal oxime (0.78 g) as a white crystal.

### (6) (E)-7-Phenyl-7-(3-pyridyl)-6-hepten-1-yl hydroxylamine

Under Ar, sodium cyanoborohydride (0.88 mg, 13.98 mmol) was added to an acetic acid (4.0 ml) solution of (E)-7-phenyl-7-(3-pyridyl)-6-heptenal oxime (0.78 g, 2.80 mmol) at 0°C and the mixture was stirred at room temperature for 15 minutes. After the reaction, a pH of the solution was adjusted to a pH of 7 with a 15% aqueous sodium hydroxide solution, then made a pH of at least 9 with an aqueous saturated sodium hydrogencarbonate solution and extracted with ethyl acetate. The organic phase was washed with brine and dried over magnesium sulfate, then the solvent was evaporated in vacuo and the residue was purified by silica gel column chromatography to obtain (E)-7-phenyl-7-(3-pyridyl)-6-hepten-1-yl hydroxylamine (0.62 g) as a white crystal.

### (7) N-Hydroxy-N-[(E)-7-phenyl-7-(3-pyridyl)-6-hepten-1-yl]urea

Under Ar, trimethylsilyl isocyanate (0.36 ml, 2.65 mmol) was added to a tetrahydrofuran (8.0 ml) solution of (E)-7-phenyl-7-(3-pyridyl)-6-hepten-1-ylhydroxylamine (623 mg, 2.209 mmol), then the mixture was stirred at room temperature for 15 hours. After the reaction, the volatile material was evaporated in vacuo and the residue was purified by silica gel column chromatography to obtain N-hydroxy-N-[(E)-7-phenyl-7-(3-pyridyl)-6-hepten-1-yl] urea (552 mg) as a white crystal.
¹H-NMR (CDCl₃): δ 1.32 (m, 2H), 1.44 (m, 2H), 1.60 (m, 2H), 2.13 (m, 2H), 3.52 (t, 2H), 6.08 (t, 1H), 7.12 (m, 1H), 7.17 (m, 1H), 7.26-7.40 (m, 5H), 8.37 (m, 1H), 8.54 (d, 1H).

### Example 2

### Synthesis of N-hydroxy-N-[(Z)-7-phenyl-7-(3-pyridyl)-6-hepten-1-yl]urea (Compound 2)

The above compound was synthesized by the same method as in Example 1, using the (Z)-7-phenyl-7-(3-pyridyl)-6-heptenoate ethyl ester obtained in Example 1 (2).
¹H-NMR (CDCl₃): δ 1.32 (m, 2H), 1.47 (m, 2H), 1.56 (m, 2H), 2.05 (m, 2H), 3.51 (t, 2H), 5.46 (br, 2H), 6.15 (t, 1H), 7.16 (m, 2H), 7.19-7.29 (m, 4H), 7.33 (m, 1H), 7.49 (m, 1H), 8.43 (d, 1H), 8.47 (m, 1H).

### Example 3

### Synthesis of N'-cyclohexyl-N-hydroxy-N-[(Z)-7-phenyl-7-(3-pyridyl)-6-hepten-1-yl]urea (Compound 3)

The above compound was synthesized using cyclohexylisocyanate instead of the trimethylsilyl isocyanate in Example 2.
¹H-NMR (CDCl₃): δ 1.14-2.04 (m, 18H), 4.10 (m, 2H), 4.18-4.20 (m, 1H), 6.20 (m, 1H), 6.98 (d, 1H), 7.14-7.29 (m, 5H), 7.38 (m, 1H), 7.57 (m, 1H), 8.33 (d, 1H), 8.40 (m, 1H).

### Example 4

### Synthesis of N-hydroxy-N-[4-(3-pyridyl)-4-(3-trifluoromethylphenyl)-3-buten-1-yl] urea (Compound 4)

### (1) 1-(3-Pyridyl)-1-(3-trifluoromethylphenyl)-4-trimethylsilyloxybutan-1-ol

Under Ar, magnesium (1.67 g, 69.583 mmol) and iodine (catalytic amount) were added to a tetrahydrofuran (96 ml) solution of m-bromobenzotrifluoride (7.8 g, 34.665 mmol). This was gradually heated to prepare a Grignard reagent. Next, a tetrahydrofuran (56 ml) solution of 1-(3-pyridyl)-4-trimethylsilyloxybutan-1-one (3.91 g, 17.378 mmol) was added dropwise at 0°C, then the mixture stirred at room temperature for 1 hour. After the reaction, water was added dropwise and the reaction mixture was extracted with ethyl acetate. The organic phase was washed with brine, then was dried over magnesium sulfate. The solvent was evaporated in vacuo to obtain a crude product of 1-(3-pyridyl)-1-(3-trifluoromethylphenyl)-4-trimethylsilyloxybutan-1-ol (5.69 g). This was used for the next reaction without purification.

### (2) 1-(3-Pyridyl)-1-(3-trifluoromethylphenyl)butan-1,4-diol

1-(3-Pyridyl)-1-(3-trifluoromethylphenyl)-4-trimethylsilyloxybutan-1-ol (5.69 g) was dissolved in ethanol (40 ml), then 10% HCl (10 ml) was added dropwise and the mixture was stirred at room temperature for 1 hour. After the reaction, an aqueous saturated sodium hydrogencarbonate solution was added, the solvent was concentrated in vacuo, then the residue was extracted with ethyl acetate. The organic phase was washed with brine and dried over magnesium sulfate, then the solvent was evaporated in vacuo to obtain a crude product of 1-(3-pyridyl)-1-(3-trifluoromethylphenyl) butan-1,4-diol (4.63 g).

### (3) 4-Acetoxy-1-(3-pyridyl)-1-(3-trifluoromethylphenyl)-1-butene

1-(3-Pyridyl)-1-(3-trifluoromethylphenyl)butan-1,4-diol (4.63 g) was dissolved in acetic acid (50 ml), concentrated sulfuric acid (10 ml) was dropwise added, then the mixture was stirred at 60°C for 40 minutes. After the reaction, the mixture was neutralized with sodium hydrogencarbonate, then water was added and the mixture was extracted with ethyl acetate. The resultant mixture was washed with brine, dried over magnesium sulfate, then the solvent was evaporated in vacuo and the residue was purified by silica gel column chromatography to obtain 4-acetoxy-1-(3-pyridyl)-1-(3-trifluoromethylphenyl)-1-butene (2.21 g).

### (4) 4-[3-Pyridyl)-4-(3-trifluoromethylphenyl)-3-buten-1-ol

4-Acetoxy-1-(3-pyridyl)-1-(3-trifluoromethylphenyl)-1-butene (0.89 g) was dissolved in methanol (5 ml), a 15% aqueous sodium hydroxide solution (6 ml) was added dropwise, and the mixture was stirred at room temperature for 1.5 hours. After the reaction, the solvent was concentrated in vacuo, water was added, then teh mixture was extracted with ethyl acetate. The organic phase was washed with brine and dried over magnesium sulfate, then the solvent was evaporated in vacuo to obtain 4-(3-pyridyl)-4-(3-trifluoromethylphenyl)-3-buten-1-ol. This was used for the next reaction without purification.

### (5) N,O-Bis(tert-butoxycarbonyl)-4-(3-pyridyl)-4-(3-trifluoromethylphenyl)-3-buten-1-yl hydroxylamine

4-(3-Pyridyl)-4-(3-trifluoromethylphenyl)-3-buten-1-ol (0.69 g) was reacted with N,O-bis(tert-butoxycarbonyl)hydroxyamine in accordance with the method of PCT Application No. 92/01682 under conditions of a photo Mitsunobu reaction to obtain N,O-bis(tert-butoxycarbonyl)-4-(3-pyridyl)-4-(3-trifluoromethylphenyl)-3-buten-1-yl hydroxylamine (0.98 g).

### (6) 4-(3-Pyridyl)-4-(3-trifluoromethylphenyl)-3-buten-1-ylhydroxylamine

N,O-Bis(tert-butoxycarbonyl)-4-(3-pyridyl)-4-(3-trifluoromethylphenyl)-3-buten-1-ylhydroxylamine (0.98 g) was hydrolyzed by trifluoroacetic acid in accordance with the method of PCT Application No. 92/01682 to obtain 4-(3-pyridyl)-4-(3-trifluoromethylphenyl)-3-buten-1-ylhydroxylamine (0.33 g).

### (7) N-Hydroxy-N-[4-(3-pyridyl)-4-(3-trifluoromethylphenyl)-3-buten-1-yl]urea

4-(3-Pyridyl)-4-(3-trifluoromethylphenyl)-3-buten-1-ylhydroxylamine (0.33 g) was treated by the same method as Example 1 (7) to obtain N-hydroxy-N-[4-(3-pyridyl)-4-(3-trifluoromethylphenyl)-3-buten-1-yl]urea (0.27 g).
¹H-NMR (CDCl₃):δ 2.30 (q, 2H), 3.46 (t, 3H), 6.26 (s, 2H), 6.28 (buried t, 0.5H), 6.37 (t, 0.5H), 7.31-7.74 (m, 6H), 8.39-8.58 (m, 2H), 9.21 (s, 1H)
¹³C-NMR (CDCl₃) : δ 27.60, 27.74, 49.28, 49.32, 79.37, 122.97, 123.08, 123.63, 123.90, 124.03, 124.47, 125.67, 125.98, 129.29, 129.72, 129.79, 129.95, 130.52, 131.05, 131.32, 133.94, 134.32, 134.58, 137.27, 137.38, 137.78, 139.83, 142.75, 147.93, 148.42, 148.84, 150.18, 161.66.

### Example 5

### Synthesis of N-hydroxy-N-[3-phenyl-3-(3-pyridyl) propyl]urea (Compound 5)

### (1) Ethyl β-(3-pyridyl)cinnamate

Under Ar, a tetrahydrofuran (13 ml) solution of triethylphosphonoacetate (11.7 g, 0.052 mol) was dropwise added at -30°C to a 60% tetrahydrofuran (35 ml) suspension of sodium hydride (2.1g, 0.053 mol), then the mixture was stirred at room temperature for 45 minutes. Next, a tetrahydrofuran (26 ml) solution of 3-benzoylpyridine (6 g, 0.033 mol) was dropwise added at -30°C, then the mixture was stirred at room temperature for 4 hours.

After the reaction, water was added, and the mixture was extracted with ethyl acetate. The organic phase was washed with brine and dried over magnesium sulfate, then the solvent was evaporated in vacuo and the residue was purified by silica gel column chromatography to obtain ethylβ-(3-pyridyl) cinnamate (8.63 g).

### (2) Ethyl 3-phenyl-3-(3-pyridyl)propionate

Under Ar, 10% palladium-carbon (500 mg) was added to a methanol (60 ml) solution of ethyl β-(3-pyridyl)cinnamate (8.63 g), then the mixture was shaken in a hydrogen atmosphere at room temperature at 3.5 atmospheres for 18 hours. After the reaction, the mixture was passed through a pad of Celite, then the solvent was evaporated in vacuo to obtain ethyl 3-phenyl-3-(3-pyridyl) propionate (9.27 g).

### (3) 3-Phenyl-3-(3-pyridyl)-1-propanol

Under Ar, lithium aluminum hydride (4.1 g, 0.108 mol) was added to a tetrahydrofuran (180 ml) solution of ethyl 3-phenyl-3-(3-pyridyl) propionate (9.27 g) at 0°C, then the mixture was stirred at the same temperature for 1 hour. After the reaction, water (4.1 ml), a 15% aqueous sodium hydroxide solution (4.1 ml), and water (12.3 ml) were successively added at 0°C, then the mixture was stirred at room temperature for 15 minutes. Magnesium sulfate was then added, the mixture was stirred at room temperature, the insolubles was removed by filtration, then the solvent was evaporated in vacuo and the residue was purified by silica gel column chromatography to obtain 3-phenyl-3-(3-pyridyl)-1-propanol (4.30 g).

### (4) N-Hydroxy-N-[3-phenyl-3-(3-pyridyl)propyl]urea

3-Phenyl-3-(3-pyridyl)-1-propanol (1.55 g) was used to perform the same reaction as in Example 4 (5) to (7) to obtain N-hydroxy-N-[3-phenyl-3-(3-pyridyl)propyl]urea (1.06 g).
¹H-NMR (DMSO-d₆): δ 2.25-2.30 (m, 2H), 3.21-3.26 (m, 2H), 4.07 (m, 1H), 6.30 (d, 2H), 7.16-7.34 (m, 6H), 7.72 (d, 1H),8.39 (dd, 1H), 8.54 (s, 1H), 9.31 (d, 1H).

### Example 6

### Synthesis of N'-cyclohexyl-N-hydroxy-N-[1-methyl-3-phenyl-3-(3-pyridyl) propyl]urea (Compound 6)

### (1) 3-Phenyl-3-(3-pyridyl)propanal

Under Ar, dimethylsulfoxide (3.7 ml, 0.052 mmol) was added dropwise to a dichloromethane (120 ml) solution of oxalyl chloride (2.2 ml, 0.026 mmol) at -78°C and the resultant mixture was stirred for 30 minutes.

Then, at the same temperature, a dichloromethane (14 ml) solution of the 3-phenyl-3-(3-pyridyl)-1-propanol (2.76 g, 0.013 mmol) synthesized in Example 5 (3) was dropwise added and the resultant mixture was stirred at -78°C for 1 hour. Further, triethylamine (10.9 ml, 0.078mmol) was added and the resultant mixture was stirred at 0°C for 2.5 hours. After the reaction, water was added dropwise, then the mixture was extracted with ethyl acetate and the organic phase was washed with brine, then dried over magnesium sulfate. The solvent was evaporated in vacuo, then the residue was purified by silica gel column chromatography to obtain 3-phenyl-3-(3-pyridyl)propanol (1.65 g).

### (2) 4-Phenyl-4-(3-pyridyl)-2-butanol

Under Ar, methylmagnesium bromide (1M tetrahydrofuran solution, 23.5 ml, 23.5 mol) was added dropwise to a tetrahydrofuran (53 ml) solution of 3-phenyl-3-(3-pyridyl)propanal (1.65 g, 7.83 mmol) at 0°C, then the mixture was stirred at the same temperature for 45 minutes. After the reaction, brine was added dropwise, and the mixture was extracted with ethyl acetate, then the organic phase was washed with brine, then dried over magnesium sulfate. The solvent was evaporated in vacuo, then the residue was purified by silica gel column chromatography to obtain 4-phenyl-4-(3-pyridyl)-2-butanol (1.60 g).

### (3) N'-Cyclohexyl-N-hydroxy-N-[1-methyl-3-phenyl-3-(3-pyridyl)propyl]urea

4-Phenyl-4-(3-pyridyl)-2-butanol was treated by the same method as in Example 5 (4) (using, instead of trimethylsilyl isocyanate, cyclohexyl isocyanate), to synthesize N'-cyclohexyl-N-hydroxy-N-[1-methyl-3-phenyl-3-(3-pyridyl)propyl]urea.
¹H-NMR (DMSO-d₆): δ 0.94, 0.95 (each d, 3H), 1.10-2.21(m, 12H), 3.39 (m, 1H), 3.86 (m, 1H), 4.08 (m, 1H), 6.55(d, 1H), 7.16-7.33 (m, 6H), 7.66-7.72 (m, 1H), 8.37 (dd, 1H), 8.48, 8.53 (each d, 1H), 8.90, 8.92 (each s, 1H).

### Example 7

### Synthesis of N-hydroxy-N-[4-phenyl-4-(3-pyridyl)butyl]urea (Compound 7)

### (1) 2-[3-Phenyl-3-(3-pyridyl)propyl]-1,3-dioxolane

3-Benzoylpyridine (25 g, 0.136 mol) and 2-(1,3-dioxolan-2-yl) ethyltriphenylphosphoniumbromide (120 g, 0.271 mol) were subjected to the same reaction as in Example 1 (1), then Example 5 (2) to obtain 2-[3-phenyl-3-(3-pyridyl) propyl]-1,3-dioxolane (40.1 g). This compound could also be synthesized by the method of (2).

### (2) 2-[3-Phenyl-3-(3-pyridyl)propyl]-1,3-dioxorane

Under Ar, n-butyl lithium (1.61 M, n-hexane solution, 1.86 ml, 3 mmol) was dropwise added to a tetrahydrofuran (8 ml) solution of N,N-diisopropylamine (0.42 ml, 3 mmol) at -78°C, then the mixture was stirred at 0°C for 30 minutes. This was again cooled to -78°C, then a tetrahydrofuran solution (0.5 ml) of 3-benzylpyridine (0.49 ml, 3 mmol) was added dropwise and the mixture was stirred at 0°C for 30 minutes. The resultant mixture was again cooled to -78°C, then 2-(2-bromoethyl)-1,3-dioxolane (0.35 ml, 3 mmol) was added dropwise and the mixture was stirred at the same temperature for 1 hour and then at 0°C for 10 minutes. After the reaction, a saturated aqueous solution of ammonium chloride was added dropwise and the mixture was extracted with ethyl acetate. The organic phase was washed with brine and dried over magnesium sulfate, then the solvent was evaporated in vacuo and the residue was purified by silica gel column chromatography to obtain 2-[3-phenyl-3-(3-pyridyl)propyl]-1,3-dioxolane (601 mg).

### (3) 4-Phenyl-4-(3-pyridyl) butanal

A 10% aqueous hydrochloric acid solution (350 ml) was added at 0°C to a tetrahydrofuran (1 liter) solution of 2-[3-phenyl-3-(3-pyridyl)propyl]-1,3-dioxolane (36.39 g, 13.51 mol) and the mixture was stirred at room temperature for 4 hours. After the reaction, an aqueous concentrated potassium hydroxide solution was dropwise added at 0°C to make the solution alkali and the mixture was extracted with ethyl acetate. The organic phase was washed with brine and dried over magnesium sulfate, then the solvent was evaporated in vacuo and the residue was purified by silica gel column chromatography to obtain 4-phenyl-4-(3-pyridyl) butanal (29.00 g).

### (4) N-Hydroxy-N-[4-phenyl-4-(3-pyridyl)butyl]urea

4-Phenyl-4-(3-pyridyl)butanal (2.00 g, 8.89 mmol) was used to perform the same reaction as in Example 1 (5) to (7) to obtain N-hydroxy-N-[4-phenyl-4-(3-pyridyl)butyl]urea (0.41 g).
¹H-NMR (DMSO-d₆): δ 1.36-1.43 (m, 2H), 2.00-2.06 (m, 2H), 3.31-3.36 (m, 2H), 4.01 (m, 1H), 6.20 (brs, 1H), 7.15-7.34 (m, 6H), 7.72 (m, 1H), 8.38 (m, 1H), 8.53 (d, 1H), 9.14 (s, 1H).
¹³C-NMR (DMSO-d₆): δ 25.09, 31.62, 47.78, 49.08, 123.57, 126.26, 127.59, 128.56, 134.84, 140.51, 144.33, 147.37, 149.23, 161.57.

### Example 8

### Synthesis of N-hydroxy-N'-phenyl-N-[4-phenyl-4-(3-pyridyl)butyl]urea (Compound 8)

The above compound was synthesized using, instead of the trimethylsilyl isocyanate in the final reaction of Example 7, phenylisocyanate.
¹H-NMR (DMSO-d₆): δ 1.59-1.64 (m, 2H), 2.10-2.15 (m, 2H), 3.59 (m, 2H), 3.98 (m, 1H), 7.03-7.43 (m, 11H), 7.60 (m, 1H), 8.03 (s, 1H), 8.33 (m, 1H), 8.45 (d, 1H).

### Example 9

### Synthesis of N'-cyclohexyl-N-hydroxy-N-[4-phenyl-4-(3-pyridyl)butyl]urea (Compound 9)

The above compound was synthesized using, instead of the trimethylsilyl isocyanate in the final reaction of Example 7, cyclohexylisocyanate.
¹H-NMR (DMSO-d₆): δ 1.06-1.73 (m, 12H), 1.96-2.04 (m, 2H), 3.36-3.41 (m, 3H), 3.94 (m, 1H), 5.99 (d, 1H), 7.05-7.25 (m, 6H), 7.66 (m, 1H), 8.22 (m, 1H), 8.34 (d, 1H), 9.90 (brs, 1H).

### Example 10

### Synthesis of N'-cyclohexyl-N-hydroxy-N-[4-phenyl-4-(3-pyridyl)butyl]thiourea (Compound 10)

The above compound was synthesized using, instead of the trimethylsilyl isocyanate in the final reaction of Example 7, cyclohexylisothiocyanate.
¹H-NMR (DMSO-d₆): δ 1.04-1.67 (m, 11H), 2.00-2.06 (m, 2H), 3.96-4.09 (m, 5H), 7.15-7.33 (m, 6H), 7.60 (d, 1H), 7.70 (d, 1H), 8.36 (d, 1H), 8.53 (s, 1H), 9.73 (s, 1H).

### Example 11

### Synthesis of N-hydroxy-N'-(3-methoxyphenyl)-N-[4-phenyl-4-(3-pyridyl)butyl]urea (Compound 11)

The above compound was synthesized using, instead of the trimethylsilyl isocyanate in the final reaction of Example 7, 3-methoxyphenyl isocyanate.
¹H-NMR (CDCl₃): δ 1.51-1.56 (m, 2H), 1.99-2.05 (m, 2H), 3.62 (m, 2H), 3.78 (m, 4H), 6.60 (m, 1H), 6.91-7.26 (m, 9H), 7.56 (m, 1H), 8.09 (s, 1H), 8.29 (m, 2H).

### Example 12

### Synthesis of N-hydroxy-N'-isopropyl-N-[4-phenyl-4-(3-pyridyl)butyl]urea (Compound 12)

The above compound was synthesized using, instead of the trimethylsilyl isocyanate in the final reaction of Example 7, isopropylisocyanate.
¹H-NMR (CDCl₃): δ 1.12-1.15 (m, 6H), 1.46-1.58 (m, 2H), 1.98-2.07 (m, 2H), 3.45-3.52 (m, 2H), 3.83 (m, 0.5H), 3.87-3.95 (m, 1H), 4.04 (m, 0.5H), 5.91 (d, 1H), 7.16-7.53 (m, 6H), 7.50 (m, 0.5H), 7.90 (d, 0.5H), 8.29 (m, 0.5H), 8.31 (d, 0.5H), 8.36 (m, 0.5H), 8.48 (s, 0.5H), 8.57 (brs, 0.5H), 9.14 (brs, 0.5H).

### Example 13

### Synthesis of N'-ethyl-N-hydroxy-N-[4-phenyl-4-(3-pyridyl)butyl]urea (Compound 13)

The above compound was synthesized using, instead of the trimethylsilyl isocyanate in the final reaction of Example 7, ethylisocyanate.
¹H-NMR (DMSO-d₆): δ 0.97 (m, 3H), 1.37-1.40 (m, 2H), 2.00-2.05 (m, 2H), 2.99-3.06 (m, 2H), 3.31-3.34 (m, 2H), 4.00 (m, 1H), 6.83 (m, 1H), 7.15-7.34 (m, 6H), 7.71 (m, 1H), 8.38 (m, 1H), 8.53 (d, 1H), 9.07 (s, 1H).

### Example 14

### Synthesis of N-hydroxy-N'-methanesulfonyl -N-[4-phenyl-4-(3-pyridyl)butyl] urea (Compound 14)

Methanesulfonylchloride (0.17 ml, 2.158 mmol) was added to a dichloromethane (10 ml) solution of N-hydroxy-N-[4-phenyl-4-(3-pyridyl)butyl]urea (615 mg, 2.158 mmol) and the mixture was stirred at room temperature for 5 minutes. After the reaction, water (5 ml) was added dropwise, the mixture was extracted with ethyl acetate, then the resultant mixture was washed with brine, dried over magnesium sulfate, then the solvent was evaporated in vacuo and the residue was purified by silica gel column chromatography to obtain N-hydroxy-N'-methanesulfonyl-N-[4-phenyl-4-(3-pyridyl)butyl]urea (697 mg).
¹H-NMR (CDCl₃): δ 1.66 (m, 2H), 2.08 (m, 2H), 3.15 (s, 3H), 3.79 (m, 2H),3.94 (t, 1H), 5.35 (br, 2H), 7.19-7.32 (m, 6H), 7.52 (m, 1H), 8.44 (m, 1H), 8.53 (d, 1H).

### Example 15

### Synthesis of N-hydroxy-N-[1-methyl-4-phenyl-4-(3-pyridyl)butyl]urea (Compound 15)

### (1) 5-Phenyl-5-(3-pyridyl)-2-pentanol

Under Ar, 4-phenyl-4-(3-pyridyl) butanal (1.91 g, 8.49 mmol) was treated in the same way as Example 6 (2) to obtain 5-phenyl-5-(3-pyridyl)-2-pentanol (1.83 g).

### (2) 5-Phenyl-5-(3-pyridyl) pentan-2-one

Jones reagent (3.4 ml) was added dropwise to an acetone (120 ml) solution of 5-phenyl-5-(3-pyridyl) pentan-2-ol (1.83 g, 7.59 mmol) and the mixture was stirred at room temperature for 30 minutes. After the reaction, isopropyl alcohol and an aqueous saturated sodium hydrogencarbonate solution were successively added at 0°C, the mixture was extracted with ethyl acetate, then the organic phase was washed with water and brine, dried over magnesium sulfate, then the solvent was evaporated in vacuo and the residue was purified by silica gel column chromatography to obtain 5-phenyl-5-(3-pyridyl) pentan-2-one (1.70 g).

### (3) N-hydroxy-N-[1-methyl-4-phenyl-4-(3-pyridyl)butyl]urea

5-Phenyl-5-(3-pyridyl)pentan-2-one was treated in the same way as Example 1 (5) to (7) to obtain N-hydroxy-N-[1-methyl-4-phenyl-4-(3-pyridyl)butyl]urea as a mixture of two diastereomers (A: 0.56 g and B: 0.28 g).
¹H-NMR (DMSO-d₆) A: δ 0.92 (m, 3H), 1.14-1.23 (m, 1H), 1.37-1.47 (m, 1H), 2.00-2.12 (m, 2H), 4.10-4.16 (m, 1H), 4.23 (m, 1H), 6.22 (d, 1H), 7.20-7.40 (m, 5H), 7.83 (m, 1H), 8.33 (d, 1H), 8.54 (d, 1H), 8.63 (d, 1H), 8.78 (d, 1H); B: δ0.92 (m, 3H), 1.12-1.19 (m, 1H), 1.39-1.45 (m, 1H), 1.93-2.00 (m, 1H), 2.01-2.07 (m, 1H), 3.96 (d, 1H), 4.07-4.14 (m, 1H), 6.22 (br, 2H), 7.17-7.33 (m, 6H), 7.70 (d, 1H), 8.37 (d, 1H), 8.51 (d, 1H), 8.78 (d, 1H).

### Example 16

### Synthesis of N-hydroxy-N-[1,4-diphenyl-4-(3-pyridyl)butyl]urea (Compound 16)

### (1) 1,4-Diphenyl-4-(3-pyridyl) butan-1-ol

Under Ar, phenyl lithium (1M n-hexane solution, 1.42 ml, 2.556 mmol) was added dropwise to a tetrahydrofuran (5 ml) solution of 4-phenyl-4-(3-pyridyl) butanal (575 mg, 2.556 mmol) at -78°C and stirred at the same temperature for 10 minutes and at 0°C for 20 minutes. After the reaction, a saturated aqueous solution of ammonium chloride was added dropwise, the mixture was extracted with ethyl acetate, then the result was washed with brine. The organic phase was dried over magnesium sulfate, then the solvent was evaporated in vacuo and the residue was purified by silica gel column chromatography to obtain 1,4-diphenyl-4-(3-pyridyl) butan-1-ol (568 mg).

### (2) 1,4-Diphenyl-4-(3-pyridyl)butan-1-one

1,4-Diphenyl-4-(3-pyridyl)butan-1-ol (537 mg) was treated in the same way as Example 15 (2) to obtain 1,4-diphenyl-4-(3-pyridyl)butan-1-one (215 mg).

### (3) N-Hydroxy-N-[1,4-diphenyl-4-(3-pyridyl)butyl]urea

1,4-Diphenyl-4-(3-pyridyl)butan-1-one (215 mg) was treated in the same way as Example 1 (5) to (7) to obtain N-hydroxy-N-[1,4-diphenyl-4-(3-pyridyl)butyl]urea (153 mg).
¹H-NMR (CDCl₃): δ 1.58-1.86 (m, 2H), 1.90-2.12 (m, 2H), 3.72 (t, 0.5H), 4.03 (t, 0.5H), 5.32 (t, 0.5H), 5.38 (br, 2H), 5.44 (m, 0.5H), 7.05-7.46 (m, 11H), 8.20 (m, 2H), 8.26 (d, 1H), 8.41 (d, 1H).

### Example 17

### Synthesis of N-hydroxy-N-[5-phenyl-5-(3-pyridyl) pentyl]urea (Compound 17)

### (1) 5-Phenyl-5-(3-pyridyl)-1-pentene

4-Phenyl-4-(3-pyridyl)butanal (5.1 g, 0.023 mmol) and methyltriphenylphosphonium bromide (13.8 g, 0.039 mmol) were used to perform the same reaction as in Example 1 (1) to obtain 5-phenyl-5-(3-pyridyl)-1-pentene (4.68 g).

### (2) 5-Phenyl-5-(3-pyridyl)-1-pentanol

Under Ar, borane dimethylsulfide (10 ml, 0.10 mmol) was added dropwise to a tetrahydrofuran (300 ml) solution of 5-phenyl-5-(3-pyridyl)-1-pentene (4.68 g, 0.021 mmol) at 0°C and the mixture was stirred at room temperature for 1 hour. Next, water, a 15% aqueous solution of sodium hydroxide, and a 30% aqueous solution of hydrogen peroxide were successively added dropwise and the mixture stirred at room temperature for 73 hours. After the reaction, a saturated solution of sodium hydrogencarbonate was added dropwise and the mixture was extracted with ethyl acetate. Next, the organic phase was washed with saturated saline and dried over magnesium sulfate, then the solvent was distilled off under reduced pressure. The residue was purified by silica gel column chromatography to obtain 5-phenyl-5-(3-pyridyl)-1-pentanol (1.58 g).

### (3) N-Hydroxy-N-[5-phenyl-5-(3-pyridyl)pentyl]urea

5-Phenyl-5-(3-pyridyl)-1-pentanol was used to perform the same reaction as in Example 4 (5) to (7) to synthesize N-hydroxy-N-[5-phenyl-5-(3-pyridyl) pentyl]urea.
¹H-NMR (DMSO-d₆): δ 1.18 (m, 2H), 1.53 (m, 2H), 2.05 (m, 2H), 3.28 (m, 2H), 3.96 (t, 1H), 6.25 (s, 2H), 7.16 (m, 1H), 7.23-7.38 (m, 5H), 7.71 (d like, 1H), 8.37 (m, 1H), 8.54 (d, 1H), 9.21 (s, 1H)

### Example 18

### Synthesis of N-hydroxy-N'-phenyl-N-[5-phenyl-5-(3-pyridyl)pentyl]thiourea (Compound 18)

5-Phenyl-5-(3-pyridyl)-1-pentanol was used to perform the same reaction as in Example 4 (5) to (7) (using, instead of trimethylsilyl isocyanate, phenylisothiocyanate) so as to synthesize N-hydroxy-N'-phenyl-N-[5-phenyl-5-(3-pyridyl)pentyl]thiourea.
¹H-NMR (DMSO-d₆): δ 1.19 (m, 2H), 1.57 (m, 2H), 2.07 (m, 2H), 3.42 (m, 2H), 4.00 (t, 1H), 7.08 (t, 1H), 7.16 (t, 1H), 7.26-7.34 (m, 11H), 7.72 (d, 1H), 8.37 (dd, 1H), 8.54 (d, 1H), 9.41 (br, 1H).

### Example 19

### Synthesis of N'-ethyl-N-hydroxy-N-[5-phenyl-5-(3-pyridyl)pentyl]urea (Compound 19)

5-Phenyl-5-(3-pyridyl)-1-pentanol was used to perform the same reaction as in Example 4 (5) to (7) (using, instead of trimethylsilyl isocyanate, ethyl isocyanate) so as to synthesize N'-ethyl-N-hydroxy-N-[5-phenyl-5-(3-pyridyl)pentyl]urea.
¹H-NMR (DMSO-d₆): δ 1.10 (t, 3H), 1.24 (m, 2H), 1.61 (m, 2H), 2.01 (m, 2H), 3.22 (m, 2H), 3.44 (q, 2H), 3.84 (t, 1H), 6.02 (t, 1H), 7.15-7.28 (m, 6H), 7.54 (d, 1H), 8.31 (d, 1H), 8.40 (s, 1H), 9.78 (br, 1H).

### Example 20

### Synthesis of N-hydroxy-N-[6-phenyl-6-(3-pyridyl)hexyl]urea (Compound 20)

### (1) 6-Phenyl-6-(3-pyridyl)-2-pentenoate methyl ester

Methyltriphenylphosphoranylidene acetate (5.46 g, 16.34 mmol) was added into a benzene (300 ml) solution of 4-phenyl-4-(3-pyridyl) butanal (3.07 g, 13.62 mmol), then mixture heated at reflux for 40 minutes. After the reaction, the solvent was evaporated in vacuo and the residue was purified by silica gel column chromatography to obtain 6-phenyl-6-(3-pyridyl)-2-pentenoate methyl ester (3.46 g).

### (2) 6-Phenyl-6-(3-pyridyl)-1-hexanol

6-Phenyl-6-(3-pyridyl)-2-pentenoate methyl ester (9.98 g, 35.46 mmol) was treated in the same way as Example 5 (2) to (3) to synthesize 6-phenyl-6-(3-pyridyl)-1-hexanol (8.22 g).

### (3) N-Hydroxy-N-[6-phenyl-6-(3-pyridyl)hexyl]urea

6-Phenyl-6-(3-pyridyl)-1-hexanol was used to perform the same reaction as in Example 4 (5) to (7) so as to obtain N-hydroxy-N-[6-phenyl-6-(3-pyridyl)hexyl]urea.
¹H-NMR (DMSO-d₆): δ 1.18 (m, 2H), 1.27 (m, 2H), 1.42 (m, 2H), 2.03 (m, 2H), 3.24 (m, 2H), 3.97 (m, 1H), 6.21 (s, 2H), 7.17 (m, 1H), 7.26-7.34 (m, 5H), 7.71 (m, 1H), 8.37 (m, 1H), 8.53 (d, 1H), 9.14 (s, 1H)

### Example 21

### Synthesis of N-hydroxy-N-[1-methyl-6-phenyl-6-(3-pyridyl)hexyl]urea (Compound 21)

### (1) 6-Phenyl-6-(3-pyridyl)hexanal

6-Phenyl-6-(3-pyridyl)-1-hexanol (3 g, 11.75 mmol) was used to perform the same reaction as in Example 6 (1) to obtain 6-phenyl-6-(3-pyridyl)hexanal (2.75 g).

### (2) N-Hydroxy-N-[1-methyl-6-phenyl-6-(3-pyridyl)hexyl]urea

6-Phenyl-6-(3-pyridyl) hexanal (1.52 g, 5.99 mmol) was used to perform the same reaction as in Example 6 (2), then Example 4 (5) to (7) to obtain N-hydroxy-N-[1-methyl-6-phenyl-6-(3-pyridyl)hexyl]urea (0.86 g).
¹H-NMR (DMSO-d₆): δ 0.93 (d, 3H), 1.16-1.44 (m, 6H), 1.99-2.05 (m, 2H), 3.96 (t, 1H), 4.00 (m, 1H), 6.18 (2H, s), 7.15-7.34 (m, 6H), 7.72 (d, 1H), 8.37 (d, 1H), 8.53 (s, 1H), 8.74 (s, 1H).

### Example 22

### Synthesis of N-hydroxy-N-[6-phenyl-6-(3-pyridyl)hexyl]thiourea (Compound 22)

6-Phenyl-6-(3-pyridyl)-1-hexanol was used to perform the same reaction as in Example 4 (5) to (7) (using, instead of trimethylsilyl isocyanate, trimethylsilyl isothiocyanate) to synthesize N-hydroxy-N-[6-phenyl-6-(3-pyridyl)hexyl]thiourea.
¹H-NMR (DMSO-d₆): δ 1.18-1.32 (m, 4H), 1.51-1.60 (m, 2H), 2.02-2.07 (m, 2H), 3.89 (t, 2H), 3.97 (t, 1H), 7.15-7.34 (m, 6H), 7.72 (d, 1H), 8.37 (dd, 1H), 8.54 (d, 1H), 9.78 (s, 1H).
¹³C-NMR (DMSO-d₆): δ 25.73, 26.00, 27.16, 34.27, 47.89, 51.83, 123.53, 126.20, 127.52, 128.51, 134.74, 140.56, 144.42, 147.29, 149.17, 178.60.

### Example 23

### Synthesis of N'-ethyl-N-hydroxy-N-[6-phenyl-6-(3-pyridyl) hexyl]urea (Compound 23)

6-Phenyl-6-(3-pyridyl)-1-hexanol was used to perform the same reaction as in Example 4 (5) to (7) (using, instead of trimethylsilyl isocyanate, ethylisocyanate) to synthesize N'-ethyl-N-hydroxy-N-[6-phenyl-6-(3-pyridyl) hexyl]urea.
¹H-NMR (DMSO-d₆): δ 0.99 (t, 3H), 1.17-1.45 (m, 6H), 2.00-2.06 (m, 2H), 3.04 (m, 2H), 3.24 (t, 2H), 3.97 (t, 1H), 6.80 (t, 1H), 7.17 (t, 1H), 7.26-7.33 (m, 5H), 7.71 (d, 1H), 8.36 (dd, 1H), 8.53 (d, 1H), 9.06 (s, 1H).

### Example 24

### Synthesis of N-hydroxy-N-[7-phenyl-7-(3-pyridyl)heptyl]urea (Compound 22)

### (1) 2-[6-Phenyl-6-(3-pyridyl)-2-hexen-1-yl]-1,3-dioxorane

4-Phenyl-4-(3-pyridyl) butanal (8.00 g) and 2-(1,3 dioxolan-2-yl) ethyltriphenylphosphoniumbromide (31 g) were treated in the same way as Example 1 (1) to obtain 2-[6-phenyl-6-(3-pyridyl)-2-hexen-1-yl]-1,3-dioxolane (9.35 g).

### (2) 7-Phenyl-7-(3-pyridyl) heptyl hydroxylamine

2-[6-Phenyl-6-(3-pyridyl)-2-hexen-1-yl]-1,3-dioxorane was used to perform the same reaction as in Example 5 (2), Example 7 (3), then Example 1 (5), (6) to obtain 7-phenyl-7-(3-pyridyl)heptyl hydroxylamine (1.82 g).

### (3) N-hydroxy-N-[7-phenyl-7-(3-pyridyl)heptyl]urea

7-Phenyl-7-(3-pyridyl) heptyl hydroxylamine (1.09 g) was used to perform the same reaction as in Example 1 (7) to obtain N-hydroxy-N-[7-phenyl-7-(3-pyridyl) heptyl]urea (0.48 g).
¹H-NMR (DMSO-d₆): δ 1.08-1.23 (m, 4H), 1.23-1.32 (m, 2H), 1.38-1.45 (m, 2H), 1.99-2.04 (m, 2H), 3.25 (t, 2H), 3.96 (t, 1H), 6.20 (s, 2H), 7.12-7.33 (m, 6H), 7.71 (d, 1H), 8.36 (m, 1H), 8.53 (d, 1H), 9.14 (s, 1H).

### Example 25

### Synthesis of N'-cyclohexyl-N-hydroxy-N-[7-phenyl-7-(3-pyridyl)heptyl]urea (Compound 25)

7-Phenyl-7-(3-pyridyl)heptyl hydroxylamine (0.46 g) was reacted using, instead of the trimethylsilyl isocyanate of Example 1 (7), cyclohexyl isocyanate to obtain N'-cyclohexyl-N-hydroxy-N-[7-phenyl-7-(3-pyridyl)heptyl]urea (0.49 g).
¹H-NMR (DMSO-d₆): δ 1.04-1.68 (m, 18H), 1.99-2.04 (m, 2H), 3.24 (t, 2H), 3.34 (m, 1H), 3.95 (t, 1H), 6.42 (d, 1H), 7.14-7.32 (m, 6H), 7.70 (d, 1H), 8.36 (m, 1H), 8.52 (d, 1H), 9.10 (s, 1H).
¹³C-NMR (DMSO-d₆): δ 24.87, 25.16, 26.06, 26.30, 27.41, 28.58, 32.92, 34.29, 47.91, 48.26, 49.98, 123.53, 126.20, 127.54, 128.53, 134.76, 140.61, 144.45, 147.33, 149.19, 159.98.

### Example 26

### Synthesis of N-hydroxy-N'-(3-methoxyphenyl)-N-[7-phenyl-7-(3-pyridyl)heptyl]urea (Compound 26)

7-Phenyl-7-(3-pyridyl)heptyl hydroxylamine (0.46 g) was reacted using, instead of the trimethylsilyl isocyanate of Example 1 (7), 3-methoxyphenyl isocyanate to obtain N-hydroxy-N'-(3-methoxyphenyl)-N-[7-phenyl-7-(3-pyridyl)heptyl]urea (0.44 g).
¹H-NMR (DMSO-d₆): δ 1.12-1.33 (m, 6H), 1.45-1.52 (m, 2H), 1.99-2.05 (m, 2H), 3.35 (t, 2H), 3.68 (s, 3H), 3.95 (t, 1H), 6.52 (m, 1H), 7.09-7.31 (m, 10H), 7.70 (d, 1H), 8.35 (m, 1H), 8.52 (d, 1H), 8.85 (s, 1H).

### Example 27

### Synthesis of N-hydroxy-N'-methanesulfonyl-N-[7-phenyl-7-(3-pyridyl)heptyl]urea (Compound 27)

N-Hydroxy-N-[7-phenyl-7-(3-pyridyl)heptyl]urea (0.29 g) was treated in the same way as Example 14 to obtain N-hydroxy-N'-methanesulfonyl -N-[7-phenyl-7-(3-pyridyl)heptyl]urea (0.33 g).
¹H-NMR (CDCl₃): δ 1.21-1.36 (m, 6H), 1.57-1.65 (m. 2H), 1.98-2.08 (m, 2H), 3.17 (s, 3H), 3.70 (br, 2H), 3.89 (t, 1H), 7.16-7.30 (m, 6H), 7.51 (d, 1H), 8.41 (m, 1H), 8.52 (d, 1H).

### Example 28

### Synthesis of N-hydroxy-N-[1-methyl-7-phenyl-7-(3-pyridyl)heptyl]urea (Compound 28)

### (1) 7-Phenyl-7-(3-pyridyl)heptanal

2-[6-Phenyl-6-(3-pyridyl)-2-hexen-1-yl]-1,3-dioxolane (2.00 g) was subjected to the same reaction as in Example 5 (2), then Example 7 (3) to obtain 7-phenyl-7-(3-pyridyl)heptanal (1.53 g).

### (2) 8-Phenyl-8-(3-pyridyl)octan-2-one

7-Phenyl-7-(3-pyridyl)heptanal (0.80 g) was treated in the same way as Example 6 (2), then Example 15 (2) to obtain 8-phenyl-8-(3-pyridyl)octan-2-one (0.64 g).

### (3) N-Hydroxy-N-[1-methyl-7-phenyl-7-(3-pyridyl)heptyl]urea

8-Phenyl-8-(3-pyridyl)octan-2-one (0.47 g) was used to perform the same reaction as in Example 1 (5) to (7) to obtain N-hydroxy-N-[1-methyl-7-phenyl-7-(3-pyridyl)heptyl]urea (0.44 g).
¹H-NMR (DMSO-d₆): δ 0.92 (d, 3H), 1.13-1.45 (m, 8H), 1.93-2.08 (m,2H), 3.95 (t, 1H), 4.02 (m, 1H), 6.17 (s, 2H), 7.13-7.33 (m, 6H), 7.70 (m, 1H), 8.36 (m, 1H), 8.52 (d, 1H), 8.73 (s, 1H).

### Example 29

### Synthesis of N-hydroxy-N'-phenyl-N-[8-phenyl-8-(3-pyridyl)octyl]thiourea (Compound 29)

### (1) 1-tert-Butyldiphenylsilyloxy-8-phenyl-8-(3-pyridyl)octane

3-Benzylpyridine (1.00 g, 5.91 mmol) and 7-tert-butyldiphenylsilyloxyheptyl-1-bromide (3.84 g, 8.86 mmol) were used to perform the same reaction as in Example 7 (2) to obtain 1-tert-butyldiphenylsilyloxy-8-phenyl-8-(3-pyridyl)octane (2.98 g).

### (2) 8-Phenyl-8-(3-pyridyl)-1-octanol

Into a tetrahydrofuran (55 ml) solution of 1-tert-butyldiphenylsilyloxy-8-phenyl-8-(3-pyridyl)octane (4.63 g, 8.87 mmol) was added tetrahydroammonium chloride (4.20 g, 13.30 mmol). The mixture was then stirred at room temperature for 1 hour. After the reaction, the mixture was diluted with water and was extracted with ethyl acetate. The organic phase was washed with brine, then was dried over magnesium sulfate and the solvent was evaporated in vacuo. The residue was purified by silica gel column chromatography to obtain 8-phenyl-8-(3-pyridyl)-1-octanol (1.40 g).

### (3) N-Hydroxy-N'-phenyl-N-[8-phenyl-8-(3-pyridyl)octyl]thiourea

8-Phenyl-8-(3-pyridyl)-1-octanol (1.32 g, 4.65 mmol) was used to perform the same reaction as in Example 4 (5) to (7) (using, instead of trimethylsilyl isocyanate, phenyl isothiocyanate) to obtain N-hydroxy-N'-phenyl-N-[8-phenyl-8-(3-pyridyl)octyl]thiourea (0.98 g).
¹H-NMR (DMSO-d₆): δ 1.15-1.64 (m, 10H), 2.02 (m,2H), 3.95 (m, 1H), 4.03 (t, 1H), 7.08-7.71 (m, 12H), 8.36 (d, 1H), 8.52 (s, 1H), 9.67 (s, 1H).

### Example 30

### Synthesis of N'-cyclohexyl-N-hydroxy-N-[1-methyl-8-phenyl-8-(3-pyridyl)octyl]urea (Compound 30)

8-Phenyl-8-(3-pyridyl)-1-octanol (1.20 g, 4.23 mmol) was used to perform the same reaction as in Example 6 (1), (2), then Example 4 (5) to (7) (using, instead of trimethylsilyl isocyanate, cyclohexyl isocyanate) to synthesize N'-cyclohexyl-N-hydroxy-N-[1-methyl-8-phenyl-8-(3-pyridyl)octyl]urea (1.19 g).
¹H-NMR (DMSO-d₆): δ 0.92 (d, 3H), 1.06-1.69 (m, 20H), 1.98-2.49 (m, 2H), 3.37 (m, 1H), 3.95 (t, 1H), 3.99 (m, 1H), 6.39 (d, 1H), 7.14-7.32 (m, 6H), 7.70 (d, 1H), 8.36 (d, 1H), 8.52 (d, 1H), 8.70 (s, 1H).

### Example 31

### Synthesis of N-hydroxy-N-[9-phenyl-9-(3-pyridyl) nonyl]urea (Compound 31)

### (1) 9-Phenyl-9-(3-pyridyl)-1-nonanol

3-Benzylpyridine (1.00 g, 5.91 mmol) and 8-tert-butyldiphenylsilyloxyoctyl-1-bromide (3.97 g, 8.86 mmol) were used to perform the same reaction as in Example 7 (2) and Example 29 (2) to obtain 9-phenyl-9-(3-pyridyl)-1-nonanol (1.56 g).

### (2) N-hydroxy-N-[9-phenyl-9-(3-pyridyl)nonyl]urea

9-Phenyl-9-(3-pyridyl)-1-nonanol (1.56 g, 5.24 mmol) was subjected to the same reaction as Example 4(5) to (7) to obtain N-hydroxy-N-[9-phenyl-9-(3-pyridyl)nonyl]urea (0.93 g).
¹H-NMR (DMSO-d₆): δ 1.17-1.44 (m, 12H), 1.98-2.03 (m, 2H), 3.28 (t, 2H), 3.95 (t, 1H), 6.22 (s, 2H), 7.13-7.32 (m, 6H), 7.69 (d, 1H), 8.36 (dd, 1H), 8.53 (d, 1H), 9.19 (s, 1H).

### Example 32

### Synthesis of N'-cyclohexyl-N-hydroxy-N-[1-methyl-9-phenyl-9-(3-pyridyl)nonyl]thiourea (Compound 32)

9-Phenyl-9-(3-pyridyl)-1-nonanol (1.26 g, 4.23 mmol) was subjected to the same reaction as Example 6 (1) to (2), then Example 4 (5) to (7) (using, instead of trimethylsilylisocyanate, cyclohexyl isothiocyanate) to synthesize N'-cyclohexyl-N-hydroxy-N-[1-methyl-9-phenyl-9-(3-pyridyl)nonyl]thiourea (1.33 g).
¹H-NMR (DMSO-d₆): δ 1.00 (d, 3H), 1.06-1.31, 1.50-1.80 (m, 22H), 1.98-2.04 (m, 2H), 3.95 (t, 1H), 4.07 (m, 1H), 5.21 (m, 1H), 7.14-7.32 (m, 5H), 7.59 (d, 1H), 7.70 (d like, 1H), 8.36 (dd, 1H), 8.52 (d, 1H), 9.30 (s, 1H).

### Example 33

### Synthesis of N-hydroxy-N-[10-phenyl-10-(3-pyridyl)decyl]urea (Compound 33)

### (1) 7-Phenyl-7-(3-pyridyl)heptanal

2-[6-Phenyl-6-(3-pyridyl)-2-hexen-1-yl]-1,3-dioxolane was subjected to the same reaction as Example 5 (2), then Example 7 (3) to obtain 7-phenyl-7-(3-pyridyl)heptanal.

### (2) 10-Phenyl-10-(3-pyridyl)decanal

7-Phenyl-7-(3-pyridyl)heptanal (3.48 g) was treated in the same way as Example 24 (1), Example 5 (2), then Example 7 (3) to obtain 10-phenyl-10-(3-pyridyl)decanal (3.19 g).

### (3) N-Hydroxy-N-[10-phenyl-10-(3-pyridyl)decyl]urea

10-Phenyl-10-(3-pyridyl) decanal (1.52 g) was treated by the same procedure as in Example 1 (5) to (7) to obtain N-hydroxy-N-[10-phenyl-10-(3-pyridyl)decyl]urea (0.49 g).
¹H-NMR (DMSO-d₆): δ 1.11-1.28 (m, 14H), 1.40-1.47 (m, 2H), 1.99-2.04 (m, 2H), 3.26 (t, 2H), 3.95 (t, 1H), 6.22 (s, 2H), 7.13-7.32 (m, 6H), 7.70 (d, 1H), 8.36 (m, 1H), 8.52 (d, 1H), 9.16 (s, 1H).
¹³C-NMR (DMSO-d₆): δ 26.24, 26.42, 27.43, 28.78, 28.86, 28.97, 34.40, 47.97, 49.38, 123.57, 126.24, 127.56, 128.54, 134.80, 140.65, 144.47, 147.33, 149.19, 161.59.

### Example 34

### Synthesis of N-hydroxy-N-[1-methyl-10-phenyl-10-(3-pyridyl)decyl]urea (Compound 34)

### (1) 11-Phenyl-11-(3-pyridyl) undecan-2-one

10-Phenyl-10-(3-pyridyl)decanal (0.72 g) ws treated in the same way as Example 6 (2), then Example 15 (2) to obtain 11-phenyl-11-(3-pyridyl)undecan-2-one (0.67 g).

### (2) N-Hydroxy-N-[1-methyl-10-phenyl-10-(3-pyridyl)decyl]urea

11-Phenyl-11-(3-pyridyl)undecan-2-one (0.47 g) was used to perform the same reaction as in Example 1 (5) to (7) to obtain N-hydroxy-N-[1-methyl-10-phenyl-10-(3-pyridyl)decyl]urea (0.43 g).
¹H-NMR (DMSO-d₆): δ 0.94 (d, 3H), 1.10-1.28 (m, 14H), 1.39-1.47 (m, 2H), 1.99-2.04 (m, 2H), 3.95 (t, 1H), 4.01 (m, 1H), 6.17 (s, 2H), 7.13-7.33 (m, 6H), 7.70 (d, 1H), 8.36 (m, 1H), 8.52 (d, 1H), 8.74 (d, 1H).

### Example 35

### Synthesis of N'-cyclohexyl-N-hydroxy-N-[1-methyl-10-phenyl-10-(3-pyridyl)decyl]urea (Compound 35)

11-Phenyl-11-(3-pyridyl) undecan-2-one was used to perform the same procedure as in Example 1 (5) to (7) (using, instead of trimethylsilyl isocyanate, cyclohexyl isocyanate) to obtain N'-cyclohexyl-N-hydroxy-N-[1-methyl-10-phenyl-10-(3-pyridyl)decyl]urea (0.23 g).
¹H-NMR (DMSO-d₆): δ 0.93 (d, 3H), 1.04-1.72 (m, 24H), 1.99-2.04 (m, 2H), 3.38 (m, 1H), 3.95 (t, 1H), 4.01 (m, 1H), 6.39 (d, 2H), 7.13-7.32 (m, 6H), 7.70 (d, 1H), 8.36 (m, 1H), 8.52 (d, 1H), 8.72 (d, 1H).

### Example 36

### Synthesis of N-hydroxy-N-[4-(3-pyridyl)-4-(3-trifluoromethylphenyl)butyl]urea (Compound 36)

### (1) 4-(3-Pyridyl)-4-(3-trifluoromethylphenyl)-1-butanol

4-Acetoxy-1-(3-pyridyl)-1-(3-trifluoromethylphenyl)-1-butene (1.026 g, 3.063 mmol) was used to perform the same reaction as in Example 5 (2), then Example 4 (4) to obtain 4-(3-pyridyl)-4-(3-trifluoromethylphenyl)-1-butanol (1.00 g).

### (2) N-Hydroxy-N-[4-(3-pyridyl)-4-(3-trifluoromethylphenyl)butyl]urea

4-(3-Pyridyl)-4-(3-trifluoromethylphenyl)-1-butanol (1.00 g) was used to perform the same reaction as in Example 4 (5) to (7) to obtain N-hydroxy-N-[4-(3-pyridyl)-4-(3-trifluoromethylphenyl)butyl]urea (0.40 g).
¹H-NMR (DMSO-d₆): δ 1.35-1.42 (m, 2H), 2.05-2.10 (m, 2H), 4.18 (t, 1H), 6.21 (br, 2H), 7.33 (m, 1H), 7.54-7.69 (m, 4H), 7.77 (m, 1H), 8.40 (m, 1H), 8.59 (d, 1H), 9.15 (s, 1H).

### Example 37

### Synthesis of N-hydroxy-N-[4-(3-fluorophenyl)-4-(3-pyridyl)butyl]urea (Compound 37)

The above compound was synthesized by the same reaction using, instead of the m-bromobenzotrifluoride in Example 36 (1), 1-bromo-3-fluorobenzene.
¹H-NMR (CDCl₃): δ 1.45-1.58 (m, 2H), 1.93-2.04 (m, 2H), 3.47-3.63 (m, 2H), 3.88 (t, 1H), 5.61 (br, 2H), 6.83-7.23 (m, 5H), 7.46 (d, 1H), 8.30 (d, 1H), 8.42 (d, 1H), 10.54 (br, 1H).

### Example 38

### Synthesis of N-hydroxy-N-[4-(4-methoxyphenyl)-4-(3-pyridyl)butyl]urea (Compound 38)

The above compound was synthesized by the same reaction using, instead of the m-bromobenzotrifluoride in Example 36 (1), 4-bromoanisole.
¹H-NMR (CDCl₃): δ 1.48-1.56 (m, 2H), 1.92-2.01 (m, 2H), 3.48-3.60 (m, 2H), 3.81 (t, 1H), 5.60 (br, 2H), 6.80 (d, 2H), 7.06 (d, 2H), 7.16 (m, 1H), 7.45 (d, 1H), 8.26 (d, 1H), 8.40 (d, 1H).

### Example 39

### Synthesis of N-hydroxy-N-[4-(4-isopropylphenyl)-4-(3-pyridyl)butyl]urea (Compound 39)

The above compound was synthesized by the same reaction using, instead of the m-bromobenzotrifluoride in Example 36 (1), 1-bromo-4-isopropylbenzene.
¹H-NMR (DMSO-d₆): δ 1.14 (d, 6H), 1.39 (m, 2H), 2.00 (m, 2H), 2.79 (m, 1H), 3.95 (t, 1H), 6.19 (br, 2H), 7.14 (d, 2H), 7.22 (d, 2H), 7.29 (m, 1H), 7.69 (d, 1H), 8.36 (m, 1H), 8.51 (d, 1H), 9.14 (s, 1H).

### Example 40

### Synthesis of N-hydroxy-N-[(E)-7-phenyl-7-(3-pyridyl)-2-hepten-1-yl]urea (Compound 40)

### (1) (E)-7-Phenyl-7-(3-pyridyl)-2-heptenal

2-[6-Phenyl-6-(3-pyridyl)-2-hexen-1-yl]-1,3-dioxolane (5.00 g) was used to perform the same procedure as in Example 7 (3) to obtain (E)-7-phenyl-7-(3-pyridyl)-2-heptenal (3.63 g).

### (2) (E)-7-Phenyl-7-(3-pyridyl)-2-hepten-1-ol

(E)-7-Phenyl-7-(3-pyridyl)-2-heptenal (1.50 g, 5.65 mmol) was dissolved in methanol (15 ml), sodium borohydride (0.26 g, 6.90 mmol) was added, and the mixture was stirred at room temperature for 15 minutes. After the reaction, a saturated aqueous solution of ammonium chloride was added dropwise, the mixture was extracted with ethyl acetate, then the organic phase was washed with brine. This was dried over magnesium sulfate, the solvent was evaporated in vacuo, then the residue was purified by silica gel column chromatography to obtain (E)-7-phenyl-7-(3-pyridyl)-2-hepten-1-ol (1.40 g).

### (3) N-Hydroxy-N-[(E)-7-phenyl-7-(3-pyridyl)-2-hepten-1-yl]urea

(E)-7-Phenyl-7-(3-pyridyl)-2-hepten-1-ol (300 mg, 1.122 mmol) was used to perform the same reaction as in Example 4 (5) to (7) to obtain N-hydroxy-N-[(E)-7-phenyl-7-(3-pyridyl)-2-hepten-1-yl]urea (162 mg).
¹H-NMR (DMSO-d₆): δ 1.18-1.26 (m, 2H), 1.98-2.06 (m, 4H), 3.83 (d, 2H), 3.98 (t, 1H), 5.38 (m, 1H), 5.51 (m, 1H), 6.23 (s, 2H), 7.14-7.33 (m, 6H), 7.70 (d, 1H), 8.36 (m, 1H), 8.52 (d, 1H), 9.14 (s, 1H).

### Example 41

### Synthesis of N-hydroxy-N-[(E)-1-methyl-7-phenyl-7-(3-pyridyl)-2-hepten-1-yl]urea (Compound 41)

### (1) (E)-8-Phenyl-8-(3-pyridyl)-3-octen-2-ol

(E)-7-Phenyl-7-(3-pyridyl)-2-heptenal (1.50 g, 5.65 mmol) was used to perform the same reaction as in Example 6 (2) to obtain (E)-8-phenyl-8-(3-pyridyl)-3-octen-2-ol (1.47 g).

### (2) N-Hydroxy-N-[(E)-1-methyl-7-phenyl-7-(3-pyridyl)-2-hepten-1-yl]urea

(E)-8-Phenyl-8-(3-pyridyl)-3-octen-2-ol (0.59 g) was treated in the same way as Example 4 (5) to (7) to obtain N-hydroxy-N-[(E)-1-methyl-7-phenyl-7-(3-pyridyl)-2-hepten-1-yl]urea (0.38 g).
¹H-NMR (DMSO-d₆): δ 1.06, 1.56 (d, 3H), 1.19-1.25, 1.40-1.45 (m, 2H), 1.99-2.05 (m, 4H), 3.92-3.99 (m, 1H), 4.33-4.38, 4.53-4.59 (m, 1H), 5.35-5.47 (m, 2H), 6.18, 6.24 (each s, 2H), 7.14-7.32 (m, 6H), 7.70 (d, 1H), 8.36 (m, 1H), 8.52 (m, 1H), 8.84, 8.87 (each s, 1H).
¹³C-NMR (DMSO-d₆): δ 16.93, 27.06, 31.55, 33.83, 47.77, 53.93, 123.55, 126.20, 127.54, 128.53, 130.15, 130.50, 134.76, 140.52, 144.33, 147.31, 149.17, 161.55.

### Example 42

### Synthesis of N-hydroxy-N-[3-[α-(3-pyridyl)benzyloxy]benzyl]urea (Compound 42)

### (1) α-(3-Pyridyl)benzyl alcohol

3-Benzoylpyridine (5.00 g, 27.29 mmol) was treated in the same way as Example 40 (2) to obtain α-(3-pyridyl)benzyl alcohol (4.77 g).

### (2) α-(3-Pyridyl)benzylchloride hydrochloride

A chloroform (5 ml) solution of thionyl chloride (1.93 g, 16.20 mmol) was added dropwise to a chloroform (8 ml) solution of α-(3-pyridyl) benzyl alcohol (2.00 g, 10.80 mmol) at 0°C, then the mixture stirred at room temperature for 1 hour. After the reaction, the volatile material and the solvent was evaporated in vacuo to obtain α-(3-pyridyl) benzylchloride hydrochloride (2.79 g).

### (3) Methyl 3-[α-(3-pyridyl)benzyloxy]benzoate

Dimethylformamide (45 ml) was added to a mixture of α-(3-pyridyl) benzylchloride hydrochloride (4.17 g, 17.35 mmol), methyl 3-hydroxybenzoate (2.03 g, 13.35 mmol), and potassium carbonate (5.54 g, 40.05 mmol), then the mixture was stirred at room temperature for 1 week. After the reaction, the resultant mixture was diluted with ethyl acetate and was washed with water and brine, then dried over magnesium sulfate. The solvent was evaporated in vacuo and the residue was purified by silica gel column chromatography to obtain methyl 3-[α-(3-pyridyl)benzyloxy]benzoate (2.34 g).

### (4) 3-[α-(3-Pyridyl)benzyloxy]benzaldehyde

Methyl 3-[α-(3-pyridyl)benzyloxy]benzoate (3.82 g, 11.97 mmol) was treated in the same way as Example 5 (3), then Example 6 (1) to obtain 3-[α-(3-pyridyl) benzyloxy]benzaldehyde (3.27 g).

### (5) 3-[α-(3-Pyridyl)benzyloxy]benzylhydroxylamine

3-[α-(3-Pyridyl)benzyloxy]benzaldehyde (1.61 g, 5.56 mmol) was subjected to the same reaction as Example 1 (5), (6) to obtain 3-[α-(3-pyridyl) benzyloxy]benzylhydroxylamine (1.86 g).

### (6) N-Hydroxy-N-[3-[α-(3-pyridyl)benzyloxy]benzyl]urea

3-[α-(3-Pyridyl)benzyloxy]benzyl hydroxylamine (1.05 g) was treated in the same way as in Example 1 (7) to obtain N-hydroxy-N-[3-[α-(3-pyridyl) benzyloxy]benzyl]urea (0.80 g).
¹H-NMR (DMSO-d₆): δ 4.46 (s, 2H), 6.30 (s, 2H), 6.60 (s,1H), 6.82-7.57 (m, 10H), 7.85 (m, 1H), 8.46 (d, 1H), 8.72 (s, 1H), 9.32 (d, 1H).

### Example 43

### Synthesis of N-hydroxy-N-[4-[α-(3-pyridyl)benzyloxy]benzyl]urea (Compound 43)

α-(3-Pyridyl) benzylchloride hydrochloride (0.62 g) and methyl 4-hydroxybenzoate (0.31 g) were treated in the same way as Example 42 (3) to (6) to obtain N-hydroxy-N-[4-[α-(3-pyridyl)benzyloxy)benzyl]urea (0.41 g).
¹H-NMR (DMSO-d₆): δ 4.40 (d, 2H), 6.23 (d, 2H), 6.61 (s, 1H), 6.86-7.56 (m, 10H), 7.85 (d, 1H), 8.46 (d, 1H), 8.72 (s, 1H), 9.23 (m, 1H).

### Example 44

### Synthesis of N'-cyclohexyl-N-hydroxy-N-[3-[α-(3-pyridyl)benzyloxy]benzyl]urea (Compound 44)

3-[α-(3-Pyridyl)benzyloxy]benzyl hydroxylamine (0.40g) was reacted using, instead of the trimethylsilyl isocyanate of Example 1 (7), cyclohexyl isocyanate to obtain N'-cyclohexyl-N-hydroxy-N-[3-[α-(3-pyridyl)benzyloxy]benzyl]urea (0.43 g).
¹H-NMR (DMSO-d₆): δ 1.01-1.30 (m, 5H), 1.50-1.75 (m, 5H), 3.42 (br,1H), 4.44 (s, 2H), 6.58-7.51 (m, 11H), 7.84 (d, 1H), 8.47 (br, 1H), 8.71 (s, 1H), 9.31 (s, 1H).
¹³C-NMR (DMSO-d₆): δ 24.91, 25.16, 32.86, 48.52, 53.89, 77.69, 113.68, 116.24, 120.94, 123.73, 126.42, 127.85, 128.75, 129.06, 134.14, 137.21, 140.01, 140.91, 147.97, 148.86, 156.93, 159.87.

### Example 45

### Synthesis of N-hydroxy-N'-(3-methoxyphenyl)-N-[3-[α-(3-pyridyl)benzyloxy]benzyl] urea (Compound 45)

3-[α-(3-Pyridyl)benzyloxy]benzylhydroxylamine (0.40 g) was reacted using, instead of the trimethylsilyl isocyanate of Example 1 (7), 3-methoxyphenyl isocyanate to obtain N-hydroxy-N'-(3-methoxyphenyl)-N-[3-[α-(3-pyridyl)benzyloxy]benzyl] urea (0.43 g).
¹H-NMR (DMSO-d₆): δ 3.70 (s, 3H), 4.57 (s, 2H), 6.54-7.50 (m, 16H), 7.84 (d, 1H), 8.45 (m, 1H), 8.71 (d, 1H), 8.96 (s, 1H).
¹³C-NMR (DMSO-d₆): δ 53.14, 54.90, 77.80, 104.97, 107.73, 111.50, 114.01, 116.15, 120.90, 123.72, 126.44, 127.83, 128.75, 129.15, 129.22, 134.18, 137.14, 139.54, 140.67, 140.82, 147.95, 148.86, 157.00, 157.26, 159.43.

### Example 46

### Synthesis of N-hydroxy-N-[3-[α-(3-pyridyl)benzyloxy]-α-methylbenzyl]urea (Compound 46)

### (1) 3-[α-(3-Pyridyl)benzyloxy]acetophenone

3-[α-(3-Pyridyl)benzyloxy]benzaldehyde (0.80 g) was treated in the same way as Example 6 (2), then Example 15 (2) to obtain 3-[α-(3-pyridyl)benzyloxy]acetophenone (0.75 g).

### (2) N-Hydroxy-N-[3-[α-(3-pyridyl)benzyloxy]-α-methylbenzyl]urea

3-[α-(3-Pyridyl)benzyloxy]acetophenone (0.75 g) was treated in the same way as Example 1 (5) to (7) to obtain N-hydroxy-N-[3-[α-(3-pyridyl)benzyloxy]-α-methylbenzyl]urea (0.48 g).
¹H-NMR (DMSO-d₆): δ 1.32 (d, 3H), 5.22 (m, 1H), 6.28 (s, 2H), 6.59 (s, 1H), 6.82-7.52 (m, 10H), 7.85 (d, 1H), 8.46 (m, 1H), 8.72 (d, 1H), 9.04 (s, 1H).

### Example 47

### Synthesis of N-hydroxy-N-[3-[β-(3-pyridyl)phenethyl]benzyl]urea (Compound 47)

### (1) Methyl 3-[β-(3-pyridyl)phenethyl]benzoate

3-Benzylpyridine (1.00 g, 5.91 mmol) and methyl 3-bromomethylbenzoate (1.54 g, 6.50 mmol) were reacted in the same way as in Example 7 (2) to obtain methyl 3-[β-(3-pyridyl)phenethyl]benzoate (0.47 g).

### (2) N-Hydroxy-N-[3-[β-(3-pyridyl)phenethyl]benzyl]urea

Methyl 3-[β-(3-pyridyl)phenethyl)benzoate (0.47 g) was treated in the same way as Example 42 (4) to (6) to obtain N-hydroxy-N-[3-[β-(3-pyridyl)phenethyl]benzyl]urea (0.22 g).
¹H-NMR (DMSO-d₆): δ 3.37 (d, 2H), 4.42 (m, 3H), 6.32 (s, 2H), 6.98-7.39 (m, 10H), 7.76 (d, 1H), 8.32 (m, 1H), 8.51 (d, 1H), 9.29 (s, 1H).

### Example 48

### Synthesis of N-hydroxy-N-[3-[β-(3-pyridyl)phenethyl]-α-methylbenzyl]urea (Compound 48)

### (1) 3-[β-(3-Pyridyl)phenethyl]benzaldehyde

Methyl 3-[β-(3-pyridyl)phenethyl]benzoate (0.85 g) was treated in the same way as Example 5 (3), then Example 6 (1) to obtain 3-[β-(3-pyridyl)phenetyl]benzaldehyde(0.43 g).

### (2) 3-[β-(3-Pyridyl)phenethyl]-α-methylbenzyl alcohol

3-[β-(3-Pyridyl)phenethyl] benzaldehyde (0.32 g) was treated in the same way as Example 6 (2) to obtain 3-[β-(3-pyridyl)phenethyl]-α-methylbenzyl alcohol (0.31 g).

### (3) N-Hydroxy-N-[3-[β-(3-pyridyl)phenethyl]-α-methylbenzyl]urea

3-[β-(3-Pyridyl)phenethyl]-α-methylbenzyl alcohol (0.31 g) was subjected to the same reaction as Example 4 (5) to (7) to obtain N-hydroxy-N-[3-[β-(3-pyridyl)phenethyl]-α-methylbenzyl]urea (0.19 g).
¹H-NMR (DMSO-d₆): δ 1.27 (d, 3H), 3.37 (d, 2H), 4.37 (m, 1H), 5.17 (m, 1H), 6.25 (s, 2H), 6.96-7.38 (m, 10H), 7.75 (d, 1H), 8.32 (d, 1H), 8.49 (m, 1H), 8.97 (s, 1H).

### Preparation Examples

### Example 49 (Preparation of Tablets)

| | |
|---|---|
| Compound of present invention (Compound 7) | 25 g |
| Lactose | 62 g |
| Corn starch | 40 g |
| Hydroxypropyl cellulose | 2 g |
| Magnesium stearate | 1 g |

The above compound of the present invention, lactose and corn starch were mixed until becoming homogeneous, then a 5 w/v% ethanol solution of hydroxypropyl cellulose was added and the mixture was mixed and granulated. The granules were graded by passing them through a 16 mesh sieve, then were formed into tablets by an ordinary method to obtain tablets of a weight per tablet of 130 mg, a diameter of 7 mm, and a content of the drug of 25 mg.

### Test Example 1(Test of Lipoxygenase Inhibitory Action)

Using rat polymorphonuclear leukocytes, a test of the lipoxygenase inhibitory activity of the test compounds was determined the amount of production of leukotriene B₄.

Wistar male rats (Japan Clea) were intraperitoneally administered with 12% sodium casein. After 16 hours, the peritoneal cavities were lavaged and the polymorphonuclear leukocytes were recovered. The obtained polymorphonuclear leukocytes were suspended in a phosphate buffer (137 mM NaCl, 3 mM KCl, 8 mM Na₂HPO₄, 2 mM KH₂PO₄) (2.5 × 10⁵ cells/0.4 ml), then the incubation was carried out at 37°C for 10 minutes the test compound (final concentration 10⁻⁵M) was added and then for 5 minutes after a calcium solution (10 mM CaCl₂, 0.86% NaCl) 0.1 ml was added, then 1.25 µl of calcium ionophore (20 µM, A-23187) was added and the reaction started. Five minutes after the addition, 250 µl of methanol was added to stop the reaction. After the reaction was stopped, the resultant mixture was centrifuged for 20 minutes (4°C, 3000 rpm), then the amount of leukotriene B₄ in the supernatant was measured by the EIA method (Cayman Co. kit).

As the test compound, the above mentioned Compound 1 to Compound 4, Compound 9 to Compound 11, Compound 22 to Compound 26, Compound 28, Compound 29, compound 32, Compound 33, and Compound 40 to Compound 48 were used. As the control, A-64077 was used. The activity of the compounds in inhibiting the production of leukotriene B₄ (LTBI) is shown in Table 1 by the IC₅₀.

### Test Example 2 (Test of Thromboxanease Inhibitory Action)

Human platelet microsomes were used and a test of the thromboxane synthase inhibitory activity of the compounds was determined by the amount of production of thromboxane B₂ as the thromboxane A₂ stable metabdite.

A buffer solution (20 mM tris-HCl buffer solution, 1 mM EDTA, pH 7.5) containing human platelet microsomes (50 µg protein/ml) and the test compound (final concentration of 10⁻⁶M) was pre-incubated at 0°C for 30 minutes. A reaction was initiated to added prostaglandin H₂ (100 ng/2 µl), and terminated with 1N hydrocloric acid. Then neutralized by 1M Tris-base, and centrifuged at 3000 rpm for 20 minutes. The content of the thromboxane B₂ in the supernatent was measured by the EIA method (Cayman Co. kit).

As the test compound, the same compounds as in Test Example 1 were used. As the positive control, OKY-046 was used. The activity of the compounds in suppressing production of thromboxane (TxSI) is shown in Table 1 by the IC₅₀.

From Table 1, it is seen that the compounds of the present invention have the two actions of a lipoxygenase inhibitory activity and a thromboxane synthase inhibitory activity.

**Table 1**

| | LTBI IC₅₀ (µM) | TxSI IC₅₀ (µM) |
|---|---|---|
| Compound 1 | 0.4 | 0.0009 |
| Compound 2 | 0.06 | 0.02 |
| Compound 3 | 0.05 | 0.009 |
| Compound 4 | 2 | 0.006 |
| Compound 9 | 1 | 0.0003 |
| Compound 10 | 0.3 | 0.0009 |
| Compound 11 | 0.5 | 0.002 |
| Compound 22 | 0.2 | 0.004 |
| Compound 23 | 0.2 | 0.006 |
| Compound 24 | 0.08 | 0.01 |
| Compound 25 | 0.06 | 0.005 |
| Compound 26 | 0.04 | 0.002 |
| Compound 28 | 0.1 | 0.007 |
| Compound 29 | 0.04 | 0.002 |
| Compound 32 | 0.1 | 0.009 |
| Compound 33 | 0.01 | 0.01 |
| Compound 40 | 0.2 | 0.03 |
| Compound 41 | 0.2 | 0.005 |
| Compound 42 | 0.3 | 0.06 |
| Compound 43 | 0.5 | 0.05 |
| Compound 44 | 0.2 | 0.005 |
| Compound 45 | 0.09 | 0.006 |
| Compound 46 | 1 | 0.009 |
| Compound 47 | 0.3 | 0.02 |
| Compound 48 | 0.8 | 0.03 |
| A-64077 | 1 | - |
| OKY-046 | - | 0.01 |

### Test Example 3 (Pharmacological Activity on Antigen-Induced Bronchoconstriction Model of Guinea Pig)

Guinea pigs sensitized by ovalbumin were used and orally administered with the test compound suspended in 5% gum arabic. The activity for suppressing antigen-induced bronchoconstriction after one hour after administration was measured by the Konzett-Rossler method.

Each of the sensitized guinea pigs was anesthesized by 35 mg/kg pentobarbital, a airway cannula was inserted into the trachea and connected to an artificial respirator and transducer, a vein cannula was inserted into the jugular vein, and treatment was performed by 5 mg/kg soxamethonium and 5 mg/kg mepyramine. Next, the guinea pig was inhaled a nebulized ovalbumin to cause bronchoconstriction and the activity in suppressing bronchoconstriction was found from the ratio of the amount of overflow volume to the detector to the amount of air blown volume from the artificial respirator.

As the test compound, the Compound 7 was used. As the control, the thromboxane synthase inhibitor OKY-046 and the 5-lipoxygenase inhibitor A-64077 were used. The results are shown below:

| Suppression of Respiratory Constriction | |
|---|---|
| | (ED₅₀: mg/kg) |
| Compound 7 | 70 |
| OKY-046 | 260 |
| A-64077 | 300 |

### Test Example 4 (Acute Toxicity Test)

Crj-ICR male mice (7 weeks old, five mice to a group) were used. The test compound (Compound 7) was suspended in 0.5% sodium carboxymethylcellulose and administered intraperitoneally at a ratio of 10 ml/kg. The survival of the animals was observed up until 7 days after administration.

As a result, no deaths were observed at dosages of up to 1000 mg/kg.

### INDUSTRIAL APPLICABILITY

The compounds according to the present invention have a strong inhibitory activity against lipoxygenase and thromboxane synthase, and therefore, is useful as a drug for treatment of allergic diseases or inflammatory diseases, more specifically, as a drug for the treatment or prevention of various diseases arising from the metabolites of arachidonic acid, for example, asthma, psoriasis, enteritis, nephritis, ischemia.

## Claims

1. An N-hydroxyurea derivative or a pharmacologically acceptable salt thereof having the formula (I): 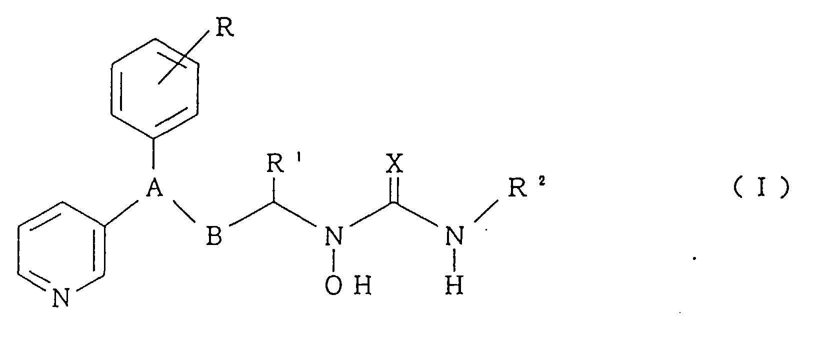 wherein, A represents CH, CH-CH₂, CH-O, or C=CH, X represents O or S, B represents a C₁ to C₈ alkylene, C₂ to C₆ alkenylene, m-phenylene, or p-phenylene, R represents a hydrogen atom, halogen atom, C₁ to C₄ alkyl which may be substituted with a halogen atom, C₁ to C₄ alkoxy which may be substituted with a halogen atom, or nitro, R¹ represents a hydrogen atom, C₁ to C₄ lower alkyl, or phenyl, and R² represents a hydrogen atom, C₁ to C₄ alkyl, cycloalkyl, phenyl which may be substituted with a substituent, or methanesulfonyl.

2. An N-hydroxyurea derivative or a pharmacologically acceptable salt thereof as claimed in claim 1, wherein, in formula (I), A represents CH and B represents a C₁ to C₈ alkylene.

3. An N-hydroxyurea derivative or a pharmacologically acceptable salt thereof as claimed in claim 1, wherein, in formula (I), A represents CH-CH₂ or CH-O and B represents a C₂ to C₄ alkenylene, m-phenylene or p-phenylene.

4. An N-hydroxyurea derivative or a pharmacologically acceptable salt thereof as claimed in claim 1, wherein, in formula (I), A represents C=CH and B represents a C₁ to C₅ alkylene.

5. An N-hydroxyurea derivative or a pharmacologically acceptable salt thereof as claimed in claim 3 or 4, wherein, in formula (I), X is O.

6. An N-hydroxyurea derivative or a pharmacologically acceptable salt thereof as claimed in any one of claims 1 to 5, wherein, in formula (I), R represents a hydrogen atom, fluorine atom, isopropyl, trifluoromethyl, or methoxy.

7. An N-hydroxyurea derivative or a pharmacologically acceptable salt thereof as claimed in claim 6, wherein, in formula (I), R is a hydrogen atom.

8. An N-hydroxyurea derivative or a pharmacologically acceptable salt thereof as claimed in any one of claims 1 to 5, wherein, in formula (I), R¹ is a hydrogen atom or a methyl.

9. An N-hydroxyurea derivative or a pharmacologically acceptable salt thereof as claimed in any one of claims 1 to 5, wherein, in formula (I), R² is a hydrogen atom, C₁ to C₄ alkyl, cycloalkyl or phenyl which may be substituted with a substituent.

10. An N-hydroxyurea derivative or a pharmacologically acceptable salt thereof as claimed in claim 9, wherein, in formula (I), R² is a hydrogen atom, ethyl, isopropyl, cyclohexyl, phenyl, or methoxyphenyl.

11. An N-hydroxyurea derivative or a pharmacologically acceptable salt thereof as claimed in claim 10, wherein, in formula (I), R² is a hydrogen atom or a cyclohexyl.

12. A pharmaceutical composition comprising an N-hydroxyurea derivative or a pharmacologically acceptable salt thereof as according to any one of claims 1 to 11 and a carrier.

13. An antiallergic or antiinflammatory pharmaceutical preparation comprising an N-hydroxyurea derivative or a pharmacologically acceptable salt thereof according to any one of claims 1 to 11, as an active ingredient, and a carrier

14. An antiasthmatic pharmaceutical preparation comprising an N-hydroxyurea derivative or a pharmacologically acceptable salt thereof according to any one of claims 1 to 11 and a carrier.
